# EUROPEAN PATENT SPECIFICATION

(11) **EP 2 417 545 B1**
(45) Date of publication and mention of the grant of the patent: **13.05.2015**
(21) Application number: 10711913.3
(22) Date of filing: 31.03.2010
(51) Int. Cl.: G06F 19/18, G06F 19/20, G06F 19/24, C12Q 1/68

(54) **METHOD FOR IN VITRO DIAGNOSING ACUTE MYELOID LEUKEMIA**
VERFAHREN ZUR IN-VITRO-DIAGNOSE VON AKUTER MYELOISCHER LEUKÄMIE
PROCEDE POUR LE DIAGNOSTIC IN VITRO DE LEUCEMIE AIGUE MYELOBLASTIQUE

(30) Priority: 07.04.2009 EP 09157517
(43) Date of publication of application: 15.02.2012
(73) Proprietor: BIOCRATES Life Sciences AG, 6020 Innsbruck (AT)
(72) Inventor: DEIGNER, Hans-Peter, 68623 Lampertheim (DE); KOHL, Matthias, 95326 Kulmbach (DE); KELLER, Matthias, 45219 Essen (DE); KOAL, Therese, A-6020 Innsbruck (AT); WEINBERGER, Klaus, A-6020 Innsbruck (AT)
(74) Representative: Simandi, Claus
(86) International application number: PCT/EP2010/054384
(87) International publication number: WO 2010/115833

(56) References cited:
- US-A1- 2007 254 295
- LANZA GIOVANNI; ET AL: "mRNA/microRNA gene expression profile in microsatellite unstable colorectal cancer" MOLECULAR CANCER, BIOMED CENTRAL, LONDON, GB, vol. 6, no. 54, 23 August 2007 (2007-08-23), pages 1-11, XP002529937 ISSN: 1476-4598
- ORESIC M; ET AL: "Phenotype Characterisation Using Integrated Gene Transcript, Protein and Metabolite Profiling" APPLIED BIOINFORMATICS, OPEN MIND JOURNALS, AUCKLAND, NZ, vol. 3, no. 4, 1 January 2004 (2004-01-01) , pages 205-217, XP008100683 ISSN: 1175-5636
- SHIN H; MARKEY M K: "A machine learning perspective on the development of clinical decision support systems utilizing mass spectra of blood samples" JOURNAL OF BIOMEDICAL INFORMATICS, ACADEMIC PRESS, NEW YORK, NY, US, vol. 39, no. 2, 1 April 2006 (2006-04-01), pages 227-248, XP024941637 ISSN: 1532-0464 [retrieved on 2006-04-01]
- GARZON RAMIRO; GAROFALO MICHELA; MARTELLI MARIA PAOLA; BRIESEWITZ ROGER; WANG LISHENG; FERNANDEZ-CYMERING CECILIA; VOLINIA STEFANO: "Distinctive microRNA signature of acute myeloid leukemia bearing cytoplasmic mutated nucleophosmin." PROCEEDINGS OF THE NATIONAL ACADEMY OF SCIENCES OF THE UNITED STATES OF AMERICA 11 MAR 2008, vol. 105, no. 10, 11 March 2008 (2008-03-11), pages 3945-3950, XP002542515 ISSN: 1091-6490 cited in the application

## Description

The present invention relates to a method for in vitro diagnosing a complex disease namely acute myeloid leukemia (AML) in accordance with claim 1 and to the use of a kit for carrying out the method in accordance with claim 5. In the following the specification is solely directed to the complex disease namely acute myeloid leukemia (AML) and other mentioned diseases are for reference only.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or detection of metabolic indicators such as glucose and the like. As far as such diseases are concerned which easily and unambiguously can be correlated with one single parameter or a few number of parameters achieved by clinical chemistry, these parameters have proved to be indispensable tools in modern laboratory medicine and diagnosis. Under the provision that excellently validated cut-off values can be provided, such as in the case of diabetes, clinical chemical parameters such as blood glucose can be reliably used in diagnosis.

In particular, when investigating pathophysiological states underlying essentially a well known pathophysiological mechanism, from which the guiding parameter is resulting, such as a high glucose concentration in blood typically reflects an inherited defect of an insulin gene, such single parameters have proved to be reliable biomarkers for "its" diseases.

However, in pathophysiological conditions, such as cancer or demyelinating diseases such as multiple sclerosis which share a lack of an unambiguously assignable single parameter or marker, differential diagnosis from blood or tissue samples is currently difficult to impossible.

In cancer prevention, screening, diagnosis, treatment and aftertreatment, it is meanwhile clinical routine to use a series of so called "tumor markers" each being somewhat specific for a certain kind of cancer to diagnose and to monitor therapy of malign processes. Such currently used tumor markers are for example Alpha-1-fetoprotein, cancer antigen 125 (CA 125), cancer antigen 15-3, CA 50, CA 72-4, carbohydrate antigen 19-9, calcitonin, carcino embryonic antigen (CEA), cytokeratine fragment 21-1, mucin-like carcinoma-associated antigen, neuron specific enolase, nuclear matrix protein 22, alkaline phosphatase, prostate specific antigen (PSA), squamous cell carcinoma antigen, telomerase, thymidine kinase, Thyreoglobulin, and tissue polypeptid antigen.

Although, in the prior art already a number of the above tumor markers are meanwhile routinely used, it very often is difficult from a single measurement to achieve a reliable diagnosis. Just by way of example, the cut-off values of the CEA is 4,6 ng/ml for non-smokers, whereas 25% of smokers show normal values in the range of 3,5 to 10 ng/ml and 1% of smokers show normal values of more than 10 ng/ml. Thus, only values above 20 ng/ml have to be interpreted as being "highly suspicious for a malign process", which leaves a significant grey zone in which the physician cannot rely upon the CEA-values measured in a patient's sample.

EP 540 573 B1 discloses similar cut-off values' problems with respect to the prostate specific antigen (PSA) in which typically total PSA is measured for diagnosing or excluding prostate cancer in a patient, and if the values are in the grey zone, it is the current approach to measure in addition to total PSA also free PSA with a monoclonal antibody assay being specific for free PSA and calculate a ratio of both parameters in order to get a more accurate approach for diagnosing prostate cancer and to differentiate from benign prostate hyperplasia.

The above examples of CEA and PSA detection impressively demonstrate what is common with all single tumor markers, namely on one hand, the relatively poor specificity, and on the other hand, uncertain and unreliable cut-off values so that the achieved values are difficult to interpret.

Thus, as a general consequence, it is recommended to consider the use of tumor markers in screening as critical. It is not rarely that increased levels of tumor markers without further clinical correlation lead to unnerving of the patients and do not have any diagnostic value at all.

Furthermore, in aftertreatment of malign diseases, it has to be noticed that every tumor marker needs a "critical mass" of cancer cells first, until it responds positively in clinical test. In addition, not every recurrent tumor must involve an increase of tumor marker levels.

In summary, single tumor markers proved to be useful in clinical practice only mostly in context with other diagnostic tools such as endoscopy and biopsy, followed by histological examination, but are not reliable in routine cancer screening.

Vis-á-vie the prior art of single tumor markers, it was a great progress to use gene expression levels of a plurality of genes with the microarray technology.

WO 2004111197A2, e.g. discloses minimally invasive sample procurement method for obtaining airway epithelial cell RNA that can be analyzed by expression profiling, e.g., by array-based gene expression profiling. These methods can be used to identify patterns of gene expression that are diagnostic of lung disorders, such as cancer, to identify subjects at risk for developing lung disorders and to custom design an array, e.g., a microarray, for the diagnosis or prediction of lung disorders or susceptibility to lung disorders. Arrays and informative genes are also disclosed for this purpose.

Such multiple gene approaches are much more reliable then the above mentioned single parameters, however, are subject to complex mathematical and bioinformatics procedures. Nevertheless, these gene expression signatures are promising tools in cancer diagnosis, but sometimes also have uncertainty limits what leads due to their underlying statistics and being restricted to one kind nucleic acids also to sometimes unreliable results and validation problems.

Staring from the above mentioned prior art, it is the problem of the present invention to provide a use of biomarkers in diagnostics tools with the highest possible sensitivity and Staring from the above mentioned prior art, it is the problem of the present invention to provide a use of biomarkers in diagnostics tools with the highest possible sensitivity and specificity for early diagnosis to identify diseased subjects, for use in patient preselection and stratification and for therapy control is a main goal in diagnostic development and still an urgent need in various complex diseases, in particular cancer.

The above problem is solved by a method in accordance with claim 1 and the use of a kit in accordance with claim 5. Discussed is a method for in vitro diagnosing a complex disease or subtypes thereof, selected from the group consisting of: cancer, in particular, acute myeloid leukemia (AML), colon cancer, kidney cancer, prostate cancer; ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis;
in at least one biological sample of at least one tissue of a mammalian subject comprising the steps of:
a) selecting at least two different species of biomolecules, wherein said species of biomolecules are selected from the group consisting of: RNA and/or its DNA counterparts, microRNA and/or its DNA counterparts, peptides, proteins, and metabolites;
b) measuring at least one parameter selected from the group consisting of presence (positive or negative), qualitative and/or quantitative molecular pattern and/or molecular signature, level, amount, concentration and expression level of a plurality of biomolecules of each species in said sample using at least two sets of different species of biomolecules and storing the obtained set of values as raw data in a database;
c) mathematically preprocessing said raw data in order to reduce technical errors being inherent to the measuring procedures used in step b);
d) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naïve Bayes; and applying said selected classifier algorithm to said preprocessed data of step c);
e) said classifier algorithms of step d) being trained on at least one training data set containing preprocessed data from subjects being divided into classes according to their pathophysiological, physiological, prognostic, or responder conditions, in order to select a classifier function to map said preprocessed data to said conditions;
f) applying said trained classifier algorithms of step e) to a preprocessed data set of a subject with unknown pathophysiological, physiological, prognostic, or responder condition, and using the trained classifier algorithms to predict the class label of said data set in order to diagnose the condition of the subject.

Dependant claims 2 to 5 are preferred embodiments of the present invention. Discussed is the use of "omics" data comprising, but not limited to mRNA expression data, microRNA expression data, proteomics data, and metabolomics data, statistical learning respectively machine learning for identification of molecular signatures and biomarkers. Examples comprise the determination of the concentrations of the aforementioned biomolecules via known methods such as polymerase chain reaction (PCR), microarrays and other methods such as sequencing to determine RNA concentrations, protein identification and quantification by mass spectrometry (MS), in particular MS-technologies such as metabolite concentrations by use of MS-technologies or alternative methods, subsequent feature selection and the combination of these features to classifiers including molecular data of at least two molecular levels (that is at least two different types of endogenous biomolecules, e.g. RNA concentrations plus metabolomics data respectively concentrations of metabolites or RNA concentrations plus concentrations of proteins or peptides etc.) and optimal composed marker sets are extracted by statistical methods and data classification methods.

The concentrations of the individual markers of the distinct molecular levels (RNA molecules, peptides/proteins, metabolites etc.) thus are measured and data processed to classifiers indicating diseased states etc. with superior sensitivities and specificities compared to procedures and biomarker confined to one type of biomolecules.

A method for the selection and combination of biomarkers and molecular signatures of biomolecules in particular utilizing one or several individual molecules of the biomolecule types mRNA, microRNA, proteins, or peptides, small endogenous compounds (metabolites) in combination (combining at least two of the aforementioned types of biomolecules), with the biomolecules obtained from body liquids or tissue, identified by use of statistical methods and classifiers derived from the data of these groups of molecules for use in diagnosis and early diagnosis, for patient stratification, therapy selection, therapy monitoring and theragnostics in complex diseases is described.

### BACKGROUND OF THE INVENTION - Prior ART

Systems biology approaches utilizing varying omics approaches such as genomics, proteomics and metabolomics are increasingly applied to research and diagnostics of complex diseases. These technologies may provide data and biological indicators, so-called (prognostic, predictive and pharmacodynamic) biomarkers with the potential to revolutionize clinical practice in diagnosis.

For early cancer detection single biomarkers are commonly used. However, the widely used cancer antigen 125 (CA125) for instance can only detect 50%-60% of patients with stage I ovarian cancer. Analogously, the single use of the prostate specific antigen (PSA) value for early stage prostate cancer identification is not specific enough to reduce the number of false positives [Petricoin EF 3rd, Ornstein DK, Paweletz CP, Ardekani A, Hackett PS, Hitt BA, Velassco A, Trucco C, Wiegand L, Wood K, Simone CB, Levine PJ, Linehan WM, Emmert-Buck MR, Steinberg SM, Kohn EC, Liotta LA, Serum proteomic patterns for detection of prostate cancer, J Natl Cancer Inst. 2002; 94(20):1576-8.] and it is evident that it is highly unlikely that a complex disease can be characterized or diagnosed and the effect of therapies assessed by use of single biomarkers.

Recent advances in diagnostic tools e.g. in cancer diagnostics typically comprise multicomponent tests utilizing several biomarkers of the same class of biomolecules such as several proteins, RNA or microRNA species and the analysis of high dimensional data gives a deeper insight into the abnormal signaling and networking which has a high potential to identify previously not discovered marker candidates. However, methods according to the present state of the art utilize single biomolecules or sets of a single type of biomolecules for biomarkers sets such as several RNA, microRNA or protein molecules. See Garzon R, Volinia S, Liu CG, Fernandez-Cymering C, Palumbo T, Pichiorri F, Fabbri M, Coombes K, Alder H, Nakamura T, Flomenberg N, Marcucci G, Calin GA, Kornblau SM, Kantarjian H, Bloomfield CD, Andreeff M, Croce CM, MicroRNA signatures associated with cytogenetics and prognosis in acute myeloid leukemia, Blood. 2008; 111(6):3183-9 and Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR., Multiclass cancer diagnosis using tumor gene expression signatures. Proc Natl Acad Sci USA. 2001;98(26):15149-54. For miRNA in Cancer see WO2008055158.

In addition, Oncotype DX is an example of a recent multicomponent RNA-based test, like a multigene activity assay, to predict recurrence of tamoxifen-treated, node-negative breast cancer is disclosed in Paik S, Shak S, Tang G, Kim C, Baker J, Cronin M, Baehner FL, Walker MG, Watson D, Park T, Hiller W, Fisher ER, Wickerham DL, Bryant J, Wolmark N, Engl J Med. 2004; 351 (27):2817-26.

Habel LA, Shak S, Jacobs MK, Capra A, Alexander C, Pho M, Baker J, Walker M, Watson D, Hackett J, Blick NT, Greenberg D, Fehrenbacher L, Langholz B, Quesenberry CP describe a population-based study of tumor gene expression and risk of breast cancer death among lymph node-negative patients in Breast Cancer Res. 2006; 8(3):R25.

Other recent examples include breast-cancer gene-expression signatures — marketed for clinical use as), MammaPrint (Agendia).

Furthermore, Glas AM, Floore A, Delahaye LJ, Witteveen AT, Pover RC, Bakx N, Lahti-Domenici JS, Bruinsma TJ, Warmoes MO, Bernards R, Wessels LF, Van't Veer LJ. Disclose a method for converting a breast cancer microarray signature into a high-throughput diagnostic test in BMC Genomics. 2006; 7:278.

Another known approach is disclosed as the so called H/I test (AviaraDx), developped by Nicholas C Turner and Alison L Jones BMJ. 2008 July 19; 337(7662): 164-169, which estimates the probability of the original breast cancer recurring after it has been resected.

Although these products and prototypes demonstrate significant progress for specific areas of diagnostics, there is still an urgent need for reliable and early diagnostics with high sensitivities and specificities in a number of complex diseases such as, but not limited to, cancer, in particular, acute myeloid leukemia (AML), colon cancer, kidney cancer, prostate cancer; ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson disease. These diagnostic tools and biomarkers are also being used for the selection of responders among patients, for an assessment of disease recurrence, the selection of therapeutic options, efficacy, drug resistance and toxicity.

Data integration of various "omics" data, e.g. to identify possible alterations of protein concentrations from altered RNA transcripts is an issue familiar to systems biology and to persons skilled in the arts for years.

Despite of that, the statistical combination of biomarker sets from different types of biomolecules, independent of data integration and biochemical interpretation to combined diagnostic signatures (combining several types of biomolecules) on a statistical basis applying various classification methods as described here is not obvious, unknown to persons skilled in the art, and has not been described in the literature. It clearly is distinct to approaches utilizing an integrative multi-dimensional analysis and combining e.g. genomes, epigenomes and transcriptomes (see SIGMA2: A system for the integrative genomic multi-dimensional analysis of cancer genomes, epigenomes, and transcriptomes, Raj Chari et al. BMC Bioinformatics 2008, 9:422) which attempt to analyse biological relationships between different omics data by various means.

Essentially, the method according to the present invention combines statistically significant biomolecule parameters of at least two different types of biomolecules on a statistical basis, entirely irrespective of known or unknown biological relationship of any kind, links or apparent biological plausibility to afford a combined biomarker composed of several types of biomolecules. The patient cases underlying the invention demonstrate that a diagnostic method and disease state specific classifier composed of at least two of the aforementioned biomolecule types and those combined biomolecules of at least two types describing the respective state of cells, a tissue, an organ or an organisms best among a collective of measured molecules, is superior to a composition of molecules or markers and their delineated molecular signatures. It is further superior to classifiers of biomolecules of just one type of biomolecules and as demonstrated here yields higher sensitivities and specificities in diagnostic applications. In that, the present invention goes far beyond the current state of the art and provides a method for generating diagnostic molecular signatures affording higher sensitivities and specificities and decreased false discovery rates compared to methods available so far. The method can be applied for diagnosing various complex and completely.

### DETAILED DESCRIPTION OF THE INVENTION

### Definitions

As used herein, the term "gene expression" refers to the process of converting genetic information encoded in a gene into ribonucleic acid, RNA (e.g., mRNA, rRNA, tRNA, or snRNA) through "transcription" of the gene (i.e., via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (i.e., RNA or protein), while "down-regulation" or "repression" refers to regulation that decrease production

Polynucleotide: A nucleic acid polymer, having more than 2 bases.

"Peptides" are short heteropolymers formed from the linking, in a defined order, of α-amino acids. The link between one amino acid residue and the next is known as an amide bond or a peptide bond.

Proteins are polypeptide molecules (or consist of multiple polypeptide subunits). The distinction is that peptides are short and polypeptides/proteins are long. There are several different conventions to determine these, all of which have caveats and nuances.

A "Complex disease" is one belonging to the following group, but is not limited to this group: cancer, in particular, acute myeloid leukemia (AML), colon cancer, kidney cancer, prostate cancer; transient ischemic attack (TIA), ischemia, in particular stroke, hypoxia, hypoxic-ischemic encephalopathy, perinatal brain damage, hypoxic-ischemic encephalopathy of neotatals asphyxia; demyelinating disease, in particular, white-matter disease, periventricular leukoencephalopathy, multiple sclerosis, Alzheimer and Parkinson's disease.

Metabolite: as used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database (http://www.hmdb.ca/) and other databases and literature. This includes any substance produced by metabolism or by a metabolic process and any substance involved in metabolism.

"Metabolomics" designates the comprehensive quantitative measurement of several (2-thousands) metabolites by, but not limited to, methods such as mass spectroscopy, coupling of liquid chromatography, gas chromatography and other separation methods chromatography with mass spectroscopy.

"Oligonucelotide arrays "or" oligonucleotide chips" or "gene chips": relates to a "microarray", also referred to as a "chip", "biochip", or "biological chip", is an array of regions having a suitable density of discrete regions, e. g., of at least 100/cm², and preferably at least about 1000/cm². The regions in a microarray have dimensions, e. g. diameters, preferably in the range of between about 10-250 µm, and are separated from other regions in the array by the same distance. Commonly used formats include products from Agilent, Affymetrix, Illumina as well as spotted fabricated arrays where oligonucleotides and cDNAs are deposited on solid surfaces by means of a dispenser or manually.

It is clear to a person skilled in the art that nucleic acids, proteins and peptides as well as metabolites can be quantified by a variety of methods including the above mentioned array systems as well as, but not limited to: quantitative sequencing, quantitative polymerase chain reaction and quantitative reverse transcription polymerase chain reaction (qPCR and RT-PCR), immunoassays, protein arrays utilizing antibodies, mass spectrometry.

"microRNAs" (miRNAs) are small RNAs of 19 to 25 nucleotides that are negative regulators of gene expression. To determine whether miRNAs are associated with cytogenetic abnormalities and clinical features in acute myeloid leukemia (AML), the miRNA expression of CD34(+) cells and 122 untreated adult AML cases is evaluated using a microarray platform.

Under different species or types or classes of biomolecules in this context is understood: RNA, microRNA, proteins and peptides of various lengths as well as metabolites.

A biomarker in this context is a characteristic, comprising data of at least two biomolecules of at least two different types (RNA, microRNA, proteins and peptides, metabolites) that is measured and evaluated as an indicator of biologic processes, pathogenic processes, or responses to an therapeutic intervention. A combined biomarker as used here may be selected from at least two of the following types of biomolecules: sense and antisense nucleic acids, messenger RNA, small RNA i.e. siRNA and microRNA, polypeptides, proteins including antibodies, small endogenous molecules and metabolites.

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), self organizing maps (SOM), recursive partitioning and regression trees, K-nearest neighbor classifiers (K-NN), fuzzy classifiers, bagging, boosting, and naive Bayes and many more can be used.

The term "binding", "to bind", "binds", "bound" or any derivation thereof refers to any stable, rather than transient, chemical bond between two or more molecules, including, but not limited to, covalent bonding, ionic bonding, and hydrogen bonding. Thus, this term also encompasses hybridization between two nucleic acid molecules among other types of chemical bonding between two or more molecules.

### Description

Discussed is how biomarker data and classifier obtained by combination of at least two different types of biomolecules out of two different species of biomolecules, wherein said species of biomolecules are selected from the group consisting of: RNA and/or its DNA counterparts, microRNA and/or its DNA counterparts, peptides, proteins, and metabolites, can afford a description of a physiological state and can be used as a superior tool for diagnosing complex diseases.

The discrimination of pathological samples or tissues from healthy specimens requires a combination of data of at least two distinct types of biomolecules, a determination of their concentrations and a statistical processing and classifier generation according to the method depicted in Table 1 below.

As mentioned above a biological link between molecules combined in a biomarker by means of classification is entirely irrelevant to the outcome and selection of the issues and can not be necessarily explained by biological models.

An exemplary method comprises essentially the following steps:
**First,** a biological sample obtained from a subject or an organism is obtained.
**Second,** the amounts of biomolecules of the following types (RNA, microRNA, peptide or protein, metabolite) are measured from the biological sample and stored as raw data in a database.
**Third** the raw data from the database are preprocessed.
**Fourth,** the amount of RNA and/or its DNA counterparts, microRNA and/or its DNA counterparts, peptide or protein, metabolite detected in the sample is compared to either a standard amount of the respective biomolecule measured in a normal cell or tissue or a reference amount of the respective biomolecule stored in a database. If the amount of the biomolecules of interest in the sample is different to the amount of the biomolecules determined in the standard or control sample, the differential concentration data are processed and used for **step 5** classifier generation as described below.

The classifier is validated in **step 6** and used **in step 7**: according to the invention, the classifier utilizes data from at least two groups of biomolecules of the aforementioned types and afford a value or a score. This score is assigned to an altered physiological state of plasma, tissue or an organ with a computed probability and can indicate a diseased state, a state due to intervention (e.g. therapeutic intervention by treatment, surgery or pharmacotherapy) or an intoxication with some probability. This score can be used as a diagnostic tool to indicate that the subject or the organism is diagnosed as diseased, to indicate intoxication as having cancer.

The score and time-dependent changes of the score can be used to assess the success of a treatment or the success of a drug administered to the subject or the organism or assess the individual response of a subject or an organism to the treatment or to make a prognosis of the future course of the physiological state or the disease and the outcome. The prognoses are relative to a subject without the disease or the intoxication having normal levels or average values of the score or classifier composed of at least two biomolecules
Table 1

**Table 1: Schematic diagram of proposed method. More details are given in text.**

| **Step 1:** |
|---|
| **Biological sample obtained** |

| **Step 2:** |
|---|
| **Measurement of raw data (concentrations of biomolecules) and deposit in data base** |

| **Step 3:** |
|---|
| **Preprocessing of raw data from data base** |

| **Step 4:** |
|---|
| **Comparison to reference values and feature selection** |

| **Step 5:** |
|---|
| **Train classifier based on data of a composed biomarker composed of at least two types of biomolecules** |

| **Step 6:** |
|---|
| **Validate classifier** |

| **Step 7:** |
|---|
| **Use of the classifier to assess physiological state, as diagnostic tool to indicate a diseased state or as a prognostic tool** |

In case of mRNA and microRNA data the preprocessing of the data typically consists of background correction and normalization. The skilled person is aware of a number of suitable known background correction and normalization strategies; a comparative survey in case of Affymetrix data is given in L.M. Cope et al., A Benchmark for Affymetrix GeneChip Expression Measures, Bioinformatics 2004, 20(3), 323-331 or R.A. Irizarry et al., Comparison of Affymetrix GeneChip Expression Measures, Bioinformatics 2006, 22(7), 789-794, respectively.

Depending on the data at hand, it may also consist of some variance stabilizing transformation or transformation to normality as for instance taking the logarithm or using Box-Cox power transformations [Box, G. E. P. and Cox, D. R. An analysis of transformations (with discussion). Journal of the Royal Statistical Society B 1964, 26, 211-252].

Often also scaling e.g. by standard deviation or median absolute deviation (MAD) might be used to transform the raw data. However, this step is not necessary for all kind of data, respectively all kind of further statistical analyses and hence may also be omitted.

The feature (variable, measurement) selection step might also be optional. However, it is recommended if the number of features is larger than the number of samples. Feature selection methods try to find the subset of features with the highest discriminatory power.

Due to the high dimensionality of mRNA and microRNA data, most classification algorithms cannot be directly applied. One reason is the so-called curse of dimensionality: With increasing dimensionality the distances among the instances assimilate. Noisy and irrelevant features further contribute to this effect, making it difficult for the classification algorithm to establish decision boundaries. Further reasons why classification algorithms are not applicable on the full dimensional space are performance limitations. Ultimately, feature transformation techniques are applied before classification, e.g. in [J. S. Yu et al., Ovarian cancer identification based on dimensionality reduction for high-throughput mass spectrometry data, Bioinformatics, 21(10):2200-2209,2005]. Furthermore, also for the task of identifying unknown marker candidates, the use of traditional methods is limited due to the high dimensionality of the data.

To identify diseased subjects with the highest possible sensitivity and specificity is the main goal in diagnostic development. For this purpose, a large number of classification algorithms can be chosen e.g. logistic regression, (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more can be applied to develop new marker candidates. These algorithms are trained on at least one training data set which contains instances labeled according to classes, e.g. healthy and diseased, and then tested on at least one test data set which includes novel instances not used for the training. In the training-test step one or more rounds of cross-validation, bootstrap or some split-sample approach can be used to estimate how accurately a predictive model will perform in practice. Finally, the classifier will be used to predict the class label of novel unlabeled instances [T. M. Mitchell. Machine Learning. McGraw-Hill, 1997].

Classifiers are typically deterministic functions that map a multi-dimensional vector of biological measurements to a binary (or n-ary) outcome variable that encodes the absence or existence of a clinically-relevant class, phenotype or distinct state of disease. The process of building or learning a classifier involves two steps: (1) selection of a family functions that can approximate the systems response, and using a finite sample of observations (training data) to select a function from the family of functions that best approximates the system's response by minimizing the discrepancy or expected loss between the system's response and the function predictions at any given point.

Depending on the chosen feature selection strategy, the combination of the different data (clinical data, mRNA, microRNA, metabolites, proteins) can take place before or after feature selection. The combined data is then used as input to train and validate the classifier. However, it is also possible to train several different classifiers for the different data separately and then combine the classifiers to the predictive signature. As the data types may be very different from qualitative/categorical to quantitative/numerical, not all classifiers may work for such multilevel data; e.g., some classifiers accept only quantitative data. Hence, depending on the data types one has to choose a class of functions for classification which has an appropriate domain.

Numerous feature selection strategies for classification have been proposed, for a comprehensive survey see e.g. [M. A. Hall and G. Holmes, Benchmarking Attribute Selection Techniques for Discrete Class Data Mining. IEEE Transactions on Knowledge and Data Engineering, 15(6): 1437-1447, 2003.]. Following a common characterization, it is distinguished between filter and wrapper approaches.

Filter approaches use an evaluation criterion to judge the discriminating power of the features. Among the filter approaches, it can further be distinguished between rankers and feature subset evaluation methods. Rankers evaluate each feature independently regarding its usefulness for classification. As a result, a ranked list is returned to the user. Rankers are very efficient, but interactions and correlations between the features are neglected. Feature subset evaluation methods judge the usefulness of subsets of the features. The information of interactions between the features is in principle preserved, but the search space expands to the size of O(2<d>). For high-dimensional data, only very simple and efficient search strategies, e.g. forward selection algorithms, can be applied because of the performance limitations.

The wrapper attribute selection method uses a classifier to evaluate attribute subsets. Cross-validation is used to estimate the accuracy of the classifier on novel unclassified objects. For each examined attribute subset, the classification accuracy is determined. Adapted to the special characteristics of the classifier, in most cases wrapper approaches identify attribute subsets with higher classification accuracies than filter approaches, cf. Pochet, N., De Smet, F., Suykens, J.A., and De Moor, B.L., Systematic benchmarking of microarray data classification: assessing the role of non-linearity and dimensionality reduction. Bioinformatics, 20(17):3185-95 (2004). As the attribute subset evaluation methods, wrapper approaches can be used with an arbitrary search strategy. Among all feature selection methods, wrappers are the most computational expensive ones, due to the use of a learning algorithm for each examined feature subset.

A preferred embodiment of the present invention is a method, wherein said complex disease is AML, said mammalian subject is a human being, said biological sample blood and/or blood cells and/or bone marrow;
wherein said different species of biomolecules are microRNA and proteins, in particular surface proteins from non-mature hematopoietic stem cells, preferably CD34;
wherein microRNA expression levels and CD34 presence are used as said parameters of step b);
wherein raw data of microRNA expression are preprocessed using a variance-stabilizing normalization and summarizing the normalized multiple probe signals (technical replicates) to a single expression value, using the median;
wherein a ranker, in particular a Mann-Whitney significance test combined with largest median of pairwise differences as filter for microRNA expression data is used for said feature selection;
wherein logistic regression is selected as suitable classifying algorithm, the training of the classifying algorithm including preprocessed and filtered microRNA expression data and CD34 information (positive or negative), is carried out with an n-fold cross-validation, in particular 5 to 10-fold, preferably 5-fold cross-validation;
applying said trained logistic regression classifier to said preprocessed microRNA expression data set and CD34 information to a subject under suspicion of having AML, and using the trained classifiers to diagnose a specific AML-type.

Another example is a method, wherein said complex disease is colon cancer, said mammalian subject is a human being, said biological sample is colon tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for microRNA expression data is used for said feature selection;
wherein random forests are selected as suitable classifying algorithm, the training of the classifying algorithm including preprocessed and filtered mRNA and microRNA expression data, is carried out with a leave-one-out (LOO) cross-validation; applying said trained random forests classifier to said preprocessed mRNA and microRNA expression data sets to a subject under suspicion of having colon cancer, and using the trained classifiers to diagnose colon cancer and/or a subtype thereof.

A further example is a method, wherein said complex disease is kidney cancer, said mammalian subject is a human being, said biological sample is kidney tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Welch t-test (significance test) combined with largest mean of pairwise differences as filter for mRNA and microRNA expression data is used for said feature selection;
wherein single-hidden-layer neural networks are selected as suitable classifying algorithm, the training of the classifying algorithm including preprocessed and filtered mRNA and microRNA expression data, is carried out with a leave-one-out (LOO) cross-validation; applying said trained random forests classifier to said preprocessed mRNA and microRNA expression data sets to a subject under suspicion of having kidney cancer, and using the trained classifiers to diagnose kidney cancer and/or a subtype thereof.

Another example is a method, wherein said complex disease is prostate cancer, said mammalian subject is a human being, said biological sample is urine and/or prostate tissue;
wherein said different species of biomolecules are mRNA and/or its DNA counterparts and microRNA and/or its DNA counterparts;
wherein mRNA expression levels and microRNA expression levels are used as said parameters of step b);
wherein raw data of microRNA expression are preprocessed using a variance-stabilizing normalization;
wherein raw data of mRNA expression are preprocessed using a variance-stabilizing normalization and summarizing the perfect match (PM) and miss match (MM) probes to an expression measure using a robust multi-array average (RMA);
wherein a ranker, in particular a Mann-Whitney significance test combined with largest median of pairwise differences as filter for mRNA and microRNA expression data is used for said feature selection;
wherein linear discriminant analysis is selected as suitable classifying algorithm, the training of the classifying algorithm including preprocessed and filtered mRNA and microRNA expression data, is carried out with a leave-one-out (LOO) cross-validation;
applying said trained random forests classifier to said preprocessed mRNA and microRNA expression data sets to a subject under suspicion of having prostate cancer, and using the trained classifiers to diagnose prostate cancer and/or a subtype thereof.

Again another example is a method, wherein said complex disease is transient ischemic attack (TIA) and/or ischemia and/or hypoxia, said mammalian subject is a human being, said biological sample blood and/or blood cells and/or cerebrospinal fluid and/or brain tissue; wherein said different species of biomolecules are mRNA and/or its DNA counterparts and brain metabolites, in particular free prostaglandins, lipooxygenase derived fatty acid metabolites, glutamine, glutamic acid, leucin, alanine, serine, decosahexaenoic acid (DHA), 12(S)-hydroxyeicosatetraenoic acid (12S-HETE); wherein mRNA expression levels and quantitative and/or qualitative molecular metabolite patterns (metabolomics data) are used as said parameters of step b); wherein raw data of mRNA expression are preprocessed using action-β as reference genes and metabolomics data of said brain metabolites are preprocessed by a variance stabilizing transformation via the binary logarithm (i.e. to base 2); wherein a ranker, in particular a Welch t-test (significance test) combined with largest mean of pairwise differences as filter for metabolomics data is used for said feature selection; wherein support vector machines are selected as suitable classifying algorithm, the training of the classifying algorithm including preprocessed and filtered mRNA and microRNA expression data, is carried out with a leave-one-out (LOO) cross-validation; applying said trained support vector machines classifier to said preprocessed mRNA expression data and said metabolomics data sets to a subject under suspicion of having ischemia and/or hypoxia, and using the trained classifiers to diagnose ischemia and/or hypoxia and/or the grades thereof.

In the following the examples are solely directed to the complex disease namely acute myeloid leukemia (AML) and other mentioned diseases are for reference only.

### Examples

### Example 1 : Method utilizing microRNA and protein data

As a first example, we use the microRNA and clinical data of Garzon R, Garofalo M, Martelli MP, Briesewitz R, Wang L, Fernandez-Cymering C, Volinia S, Liu CG, Schnittger S, Haferlach T, Liso A, Diverio D, Mancini M, Meloni G, Foa R, Martelli MF, Mecucci C, Croce CM, Falini B. Distinctive microRNA signature of acute myeloid leukemia bearing cytoplasmic mutated nucleophosmin. PNAS 2008, 105(10):3945-50.

These data are available in the ArrayExpress online database http://www.ebi.ac.uk/arrayexpress under accession number E-TABM-429. Overall the microRNA data of 85 adult de novo AML patients characterized for subcellular localization/mutation status of NPM1 and FLT3 mutations are available. The hybridizations' were done using the OSU-CCC human & mouse microRNA 11 K v2 Microarray Shared Resource, Comprehensive Cancer Center, The Ohio State University (OSU-CCC).

Acute myeloid leukemia (AML) carrying NPM1 mutations and cytoplasmic nucleophosmin (NPMc+ AML) accounts for about one-third of adult AML and shows distinct features including a unique gene expression profile. The authors used microRNA expression values to distinguish NPMc+ mutated (n = 55) from the cytoplasmic-negative (NPMc-, i.e., NPM1 unmutated) cases (n = 30).

### Analysis:

For developing and validating a classifier based on these data we used logistic regression in combination with 5-fold cross-validation where each analysis step - including low level analysis - was repeated in each cross-validation step. Moreover, we repeated 5-fold cross-validation 20 times. This is one possibility. Of course, we could also have used a split-sample, a bootstrap or a different k-fold (k not equal to 5) cross-validation approach. Moreover, we could have used a different class of functions for classification e.g. (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.

The low level analysis consisted of the variance stabilizing transformation of Huber et al. (2002) [Huber W, von Heydebreck A, Sueltmann H, Poustka A, Vingron M. Variance Stabilization Applied to Microarray Data Calibration and to the Quantification of Differential Expression. Bioinformatics 2002, 18: 96-104] (often called normalization) and the averaging of the normalized replicates using the median. Again there is a large number of alternative methods which could be used. Several examples are given in L.M. Cope et al., Bioinformatics 2004, 20(3), 323-331 or R.A. Irizarry et al., Bioinformatics 2006, 22(7), 789-794. In each cross validation step we selected those five normalized and averaged microRNA probes for classification which had the largest median of pairwise differences (in absolute value) beyond those microRNA probes with p value equal or smaller than 0.01 by the Mann-Whitney test. This is, we used a so called ranker for feature selection. Again there are numerous other feature selection strategies we could have used, some examples are given in [M. A. Hall and G. Holmes. IEEE Transactions on Knowledge and Data Engineering, 15(6): 1437-1447, 2003.]. Overall a microRNA probe may have been chosen up to 100 times due to the 20 replications of the 5-fold cross-validation. We obtain the estimated errors given in Table 2.
Table 2

**Table 2: microRNA data, classification error via 5-fold cross-validation**

| **classifier vs. true** | **NPMc-** | **NPMc+** |
|---|---|---|
| **NPMc-** | 57.0% | 7.6% |
| **NPMc+** | 43.0% | 92.4% |

The estimated overall accuracy using 5-fold cross-validation is 79.9 %. In a second step we now use only those microRNA arrays where there additionally is information about CD34 (i.e., CD34 negative or CD34 positive); selecting these samples 54 NPMc+ and 29 NPMC-samples remain. Using only CD34 for classification we obtain the results given in Table 3. which corresponds to an overall accuracy of 85.5 %.
Table 3

**Table 3: CD34 data, classification error**

| **classifier vs. true** | **NPMc-** | **NPMc+** |
|---|---|---|
| **NPMc-** | 75.9% | 9.3% |
| **NPMc+** | 24.1% | 90.7% |

Now, if we combine the information of the top five microRNA probes with the CD34 information, we obtain the results given in Table 4. That is, the estimated overall accuracy using cross-validation is 88.1 %. Hence, this combination increases the overall accuracy from 79.9 % respectively, 85.5 % to 88.1 %.
Table 4

**Table 4: combination of microRNA and CD34, classification error via 5-fold cross validation**

| **classifier vs. true** | **NPMc-** | **NPMc+** |
|---|---|---|
| **NPMc-** | 80.7% | 8.0% |
| **NPMc+** | 19.3% | 92.0% |

The probes which were selected during cross-validation are given in Table 5.
Table 5

**Table 5: microRNA probes selected during 5-fold cross validation The results of the Sanger sequence search in accordance with Griffiths-Jones S, Saini HK, van Dongen S, Enright AJ. miRBase: tools for microRNA genomics, NAR 2008 36(Database Issue):D154-D158 for known human microRNAs are given in Table 6**

| **Seq-ID** | **Probe ID** | **Times selected** | **Probe sequence** |
|---|---|---|---|
| 1 | uc.124+ | 100 | TGCTCATCTGTGCACTTCTGTTCAACCTATCACACTGAGT |
| 2 | mmu-mir-335No2 | 97 | AAACCGTTTTTCATTATTGCTCCTGACCCCCTCTCATGGG |
| 3 | uc.368+A | 96 | TGCACAGGGGACCTTAACCAGATCATTAGTTTATATGCCT |
| 4 | uc.324+A | 93 | CACACACTCCAGAACAGATGGTATCCAGATGCCTTATGGG |
| 5 | uc.156+ | 74 | GCGAACCATTTCTAATGTTCTGATTTTTCAGAGCCAGCCA |
| 6 | hsa-mir-340No1 | 12 | TGTGGGATCCGTCTCAGTTACTTTATAGCCATACCTGGTA |
| 7 | uc.106+ | 6 | AGCTGAATGGTGATGGTGTGAAGTATAGGTTAAATTGGGT |
| 8 | hsa-mir-033b-prec | 4 | GTGCATTGCTGTTGCATTGCACGTGTGTGAGGCGGGTGCA |
| 9 | uc.54+A | 4 | AAAGCTGTAGGGCCTCCAGGTTCTCAAGCTGTGAGTGGAA |
| 10 | uc.85+ | 4 | TGGTTGACATATGGCTGCTAATGCCCTCCTTTCTAGTGGG |
| 11 | uc.78+A | 4 | GTGTGCGTAACGGCTGGTGTGTTTCTCTAGCTGAGCTAAT |
| 12 | mmu-mir-31No2 | 3 | ACCTGCTATGCCAACATATTGCCATCTTTCCTGTCTGACA |
| 13 | uc.195+A | 2 | ACAGTGAGTGCGAGTATTATTTCTTGCCAGCGGGTGGAAG |
| 14 | uc.7+A | 1 | ACACTGCTCGCTCTATGTTAATTTTAGCTCTTCCCCTGGA |

Table 6

**Table 6: Results of the Sanger sequence search for known human microRNAs for the microRNA probes selected during 5-fold cross validation**

| **Seq-ID** | **Probe** | **microRNA ID** | **Target sequence** |
|---|---|---|---|
| 15 | uc.124+ | hsa-mir-134 | CAGGGUGUGUGACUGGUUGACCAGAGGGCAUGCAC UGUGUUCACCCUGUGGGCCACCUAGUCACCAACCCUC |
| 16 | mmu-mir-335No2 | hsa-mir-335 | UGUUUUGAGCGGGGUCAAGAGCAAUAACGAAAAUG UUUGUCAUAAACCGUUUUUCAUUAUUGCUCCUGACCU CCUCUCAUUUGCUAUAUUCA |
| 18 | hsa-mir-340No1 | hsa-mir-340 | UUGUACCUGGUGUGAUUAUAAAGCAAUGAGACUGAUU GUCAUAUGUCGUUUGUGGGAUCCGUCUCAGUUACUUU AUAGCCAUACCUGGUAUCUUA |
| 19 | uc.106+ | hsa-mir-138-1 | CCCUGGCAUGGUGUGGUGGGGCAGCUGGUGUUGUGA AUCAGGCCGUUGCCAAUCAGAGAACGGCUACUUCACA ACACCAGGGCCACACCACACUACAGG |
| 20 | hsa-mir-033b-prec | hsa-mir-33b | GCGGGCGGCCCCGCGGUGCAUUGCUGUUGCAUUGCA CGUGUGUGAGGCGGGUGCAGUGCCUCGGCAGUGCAG CCCGGAGCCGGCCCCUGGCACCAC |
| 21 | uc.54+A | hsa-mir-339 | CGGGGCGGCCGCUCUCCCUGUCCUCCAGGAGCUCAC GUGUGCCUGCCUGUGAGCGCCUCGACGACAGAGCCG GCGCCUGCCCAGUGUCUGCGC |
| 22 | uc.85+ | hsa-mir-1976 | GCAGCAAGGAAGGCAGGGGUCCUAAGGUGUGUCCUCC UGCCCUCCUUGCUGU |
| 23 | Us.78+A | hsa-mir-223 | CCUGGCCUCCUGCAGUGCCACGCUCCGUGUAUUUGAC AAGCUGAGUUGGACACUCCAUGUGGUAGAGUGUCAGU UUGUCAAAUACCCCAAGUGCGGCACAUGCUUACCAG |
| 24 | mmu-mir-31No2 | hsa-mir-31 | GGAGAGGAGGCAAGAUGCUGGCAUAGCUGUUGAACUG GGAACCUGCUAUGCCAACAUAUUGCCAUCUUUCC |
| 25 | uc.195+A | hsa-mir-548a-3 | CCUAGAAUGUUAUUAGGUCGGUGCAAAAGUAAUUGCG AGUUUUACCAUUACUUUCAAUGGCAAAACUGGCAAUUA CUUUUGCACCAACGUAAUACUU |
| 26 | uc.7+A | hsa-mir-1912 | CUCUAGGAUGUGCUCAUUGCAUGGGCUGUGUAUAGUA UUAUUCAAUACCCAGAGCAUGCAGUGUGAACAUAAUAG AGAUU |

### Example 2.1.: mRNA and microRNA: Colon Cancer

We use the colon cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001;98(26):15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(7043):834-8] to develop a multilevel classifier using mRNA and microRNA data. The data are available from the home page of the Broad Institute [http://www.broad.mit.edu/publications/broad900 and http://www.broad.mit.edu/publications/broad993s].

Overall the mRNA and microRNA data of four normal tissues and seven tumor tissues are available. The hybridisations were done with a bead-based array containing microRNA probes as well as with the Affymetrix HU6800 and HU35KsubA array for measuring the mRNA. We used only the mRNA data of the HU6800 arrays.

### Analysis:

For developing and validating a classifier based on these data we used random forests [Breiman, L. Random Forests, Machine Learning 2001, 45(1), 5-32] in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.

The preprocessing (also called low level analysis) consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006). In each cross validation step we selected those six normalized microRNA probes, respectively those six normalized mRNA probes for classification which had the largest median of pairwise differences (in absolute value) beyond those probes with p value equal or smaller than 0.1 by the Mann-Whitney test. This is, we used a so called ranker for feature selection. Again there are numerous other feature selection strategies we could have used some examples are given in [M. A. Hall and G. Holmes. IEEE Transactions on Knowledge and Data Engineering, 15(6): 1437-1447, 2003.]. Overall a microRNA, respectively mRNA probe may have been chosen up to eleven times due to LOO cross-validation.

Using only microRNA data we obtain the estimated errors given in Table 7
Table 7

**Table 7: microRNA data, classification error via leave-one-out cross validation**

| **classifier vs. true** | **colon cancer** | **normal** |
|---|---|---|
| **colon cancer** | 85.7% | 0.0% |
| **normal** | 14.3% | 100.0% |

That is, we observe a sensitivity of 85.7% and a specificity of 100.0%. The positive predictive value is equal to 100.0%, the negative predictive value is equal to 80%. The estimated overall accuracy using LOO cross-validation is 90.9 %. In a second step we used the mRNA data of the HU6800 array. The results can be read off from Table 8. We get an estimated overall accuracy of 72.7 % again using LOO cross-validation. The estimated sensitivity is equal to 85.7%, the estimated specificity is equal to 50%, the estimated positive predictive value is equal to 75.0%, the estimated negative predictive value is equal to 66.7%.
Table 8

**Table 8: mRNA data, classification error via leave-one-out cross validation**

| **classifier vs. true** | **colon cancer** | **normal** |
|---|---|---|
| **colon cancer** | 85.7% | 50.0% |
| **normal** | 14.3% | 50.0% |

In the last step we combined microRNA and mRNA data and obtained the results given in Table 9. That is, the estimated overall accuracy using cross-validation is 100.0 %. Hence, this combination increases the overall accuracy from 90.9 % respectively, 72.7 % to 100.0 %. Likewise sensitivity, specificity, positive predictive value and negative predictive value increase to 100%.
Table 9

**Table 9: microRNA and mRNA data, classification error via leave-one-out cross validation**

| **classifier vs. true** | **colon cancer** | **normal** |
|---|---|---|
| **colon cancer** | 100.0% | 0.0% |
| **normal** | 0.0% | 100.0% |

The microRNA probes which were selected during cross-validation are given in Table 10.
Table 10

**Table 10: microRNA probes selected during leave-one-out cross validation**

| **Seq-ID** | **Probe ID** | **Times selected** | **Probe sequence** |
|---|---|---|---|
| 27 | hsa-miR-1 | 11 | ATACATACTTCTTTACATTCCA |
| 28 | mmu-miR-10b | 11 | ACACAAATTCGGTTCTACAGGG |
| 29 | hsa-miR-195 | 11 | GCCAATATTTCTGTGCTGCTA |
| 30 | hsa-miR-133a | 11 | ACAGCTGGTTGAAGGGGACCAA |
| 31 | hsa-miR-135b | 10 | CACATAGGAATGAAAAGCCATA |
| 32 | hsa-miR-182 | 7 | TGTGAGTTCTACCATTGCCAAA |
| 33 | hsa-miR-30e | 4 | TCCAGTCAAGGATGTTTACA |
| 34 | hsa-miR-99a | 1 | CACAAGATCGGATCTACGGGT |

The results of the Sanger sequence search (see Griffiths-Jones S, Saini HK, van Dongen S, Enright AJ. miRBase: tools for microRNA genomics. NAR 2008 36(Database Issue):D154-D158) for known human microRNAs are given in Table 11
Table 11.

**Table 11: Results of the Sanger sequence search for known human microRNAs or the microRNA probes selected during 5-fold cross validation**

| **Seq-ID** | **Probe ID** | **microRNA ID** | **Target sequence** |
|---|---|---|---|
| 35 | hsa-miR-1 | hsa-mir-1 | ACCUACUCAGAGUACAUACUUCUUUAUGUACCCAUAUGAA CAUACAAUGCUAUGGAAUGUAAAGAAGUAUGUAUUUUUGG UAGGC |
| 36 | mmu-miR-10b | hsa-mir-10b | CCAGAGGUUGUAACGUUGUCUAUAUACCCUGUAGAACC GAAUUUGUGUGGUAUCCGUAUAGUCACAGAUUCGAUUCUA GGGGAAUAUAUGGUCGAUGCAAAAACUUCA |
| 37 | hsa-miR-195 | hsa-mir-195 | AGCUUCCCUGGCUCUAGCAGCACAGAAAUAUUGGCACAGG GAAGCGAGUCUGCCAAUAUUGGCUGUGCUGCUCCAGGCA GGGUGGUG |
| 38 | hsa-miR-133a | hsa-mir-133a | ACAAUGCUUUGCUAGAGCUGGUAAAAUGGAACCAAAUCGC CUCUUCAAUGGAUUUGGUCCCCUUCAACCAGCUGUAGCUA UGCAUUGA |
| 39 | hsa-miR-135b | hsa-mir-135b | CACUCUGCUGUGGCCUAUGGCUUUUCAUUCCUAUGUGAU UGCUGUCCCAAACUCAUGUAGGGCUAAAAGCCAUGGGCUA CAGUGAGGGGCGAGCUCC |
| 40 | hsa-miR-182 | hsa-mir-182 | GAGCUGCUUGCCUCCCCCGUUUUGGCAAUGGUAGAAC UCACACUGGUGAGGUAACAGGAUCCGGUGGUUCUAGACU UGCCAACUAUGGGGCGAGGACUCAGCCGGCAC |
| 41 | hsa-miR-30e | hsa-mir-30e | GGGCAGUCUUUGCUACUGUAAACAUCCUUGACUGGAAGCU GUAAGGUGUUCAGAGGAGCUUUCAGUCGGAUGUUUACAG CGGCAGGCUGCCA |
| 42 | hsa-miR-99a | hsa-mir-99a | CCCAUUGGCAUAAACCCGUAGAUCCGAUCUUGUGGUGAAG UGGACCGCACAAGCUCGCUUCUAUGGGUCUGUGUCAGUG UG |

The mRNA probes which were selected during cross-validation are given in Table 12. The probe sequences were obtained from Bioconductor package hu6800probe [The Bioconductor Project, www.bioconductor.org (2008). hu6800probe: Probe sequence data for microarrays of type hu6800. R package version 2.2.0.].
Table 12

Miss match (MM) probes are obtained by altering the medium amino acid, more precise A becomes T, T becomes A, G becomes C and C becomes G. The probe sequences each have a lenght of 25, i.e. the respective 13. amino acids are replaced.

**Table 12: mRNA probes selected during leave-one-out cross validation**

| **Seq-ID** | **Affymetrix ID** | **Times selected** | **Probe Sequences (Perfect Match)** |
|---|---|---|---|
| 43-62 | AFFX-HSAC07/X00351 M at | 11 | [1] AAGATCATTGCTCCTCCTGAGCGCA |
| | | | [2] CCTCCTGAGCGCAAGTACTCCGTGT |
| | | | [3] TCCGTGTGGATCGGCGGCTCCATCC |
| | | | [4] CAGATGTGGATCAGCAAGCAGGAGT |
| | | | [5] GTCCACCGCAAATGCTTCTAGGCGG |
| | | | [6] ACCACGGCCGAGCGGGAAATCGTGC |
| | | | [7] CTGTGCTACGTCGCCCTGGACTTCG |
| | | | [8] GAGCAAGAGATGGCCACGGCTGCTT |
| | | | [9] TCCTCCCTGGAGAAGAGCTACGAGC |
| | | | [10] CTGCCTGACGGCCAGGTCATCACCA |
| | | | [11] CAGGTCATCACCATTGGCAATGAGC |
| | | | [12] CGGTTCCGCTGCCCTGAGGCACTCT |
| | | | [13] CCTGAGGCACTCTTCCAGCCTTCCT |
| | | | [14] GAGTCCTGTGGCATCCACGAAACTA |
| | | | [15] ATCCACGAAACTACCTTCAACTCCA |
| | | | [16] AACTCCATCATGAAGTGTGACGTGG |
| | | | [17] GACATCCGCAAAGACCTGTACGCCA |
| | | | [18] AACACAGTGCTGTCTGGCGGCACCA |
| | | | [19] ACCATGTACCCTGGCATTGCCGACA |
| | | | [20] CAGAAGGAGATCACTGCCCTGGCAC |
| 63-81 | X03689_s_at | 10 | [1] AGATTCGGGCAAGTCCACCACTACT |
| | | | [2] TTCGGGCAAGTCCACCACTACTGGC |
| | | | [3] CACCACTACTGGCCATCTGATCTAT |
| | | | [4] CCATCTGATCTATAAATGCGGTGGC |
| | | | [5] TCTGATCTATAAATGCGGTGGCATC |
| | | | [6] TGCCTGGGTCTTGGATAAACTGAAA |
| | | | [7] TGAAAGCTGAGCGTGAACGTGGTAT |
| | | | [8] CGTGAACGTGGTATCACCATTGATA |
| | | | [9] GAACGTGGTATCACCATTGATATCT |
| | | | [10] GTGGTATCACCATTGATATCTCCTT |
| | | | [11] TATCACCATTGATATCTCCTTGTGG |
| | | | [12] CCATTGATATCTCCTTGTGGAAATT |
| | | | [13] GTACTATGTGACTATCATTGATGCC |
| | | | [14] CTATGTGACTATCATTGATGCCCCA |
| | | | [15] CTCATATCAACATTGTCGTCATTGG |
| | | | [16] TATCAACATTGTCGTCATTGGACAC |
| | | | [17] CATTGTCGTCATTGGACACGTAGAT |
| | | | [18] TGTCGTCATTGGACACGTAGATTCG |
| | | | [19] CGTCATTGGACACGTAGATTCGGGC |
| 82-101 | AFFX-HSAC07/X00351_5_at | 9 | [1] GGGTCAGAAGGATTCCTATGTGGGC |
| | | | [2] GAAGGATTCCTATGTGGGCGACGAG |
| | | | [3] CCCCATCGAGCACGGCATCGTCACC |
| | | | [4] CGTCACCAACTGGGACGACATGGAG |
| | | | [5] CACCTTCTACAATGAGCTGCGTGTG |
| | | | [6] TCCCGAGGAGCACCCCGTGCTGCTG |
| | | | [7] GGCCAACCGCGAGAAGATGACCCAG |
| | | | [8] CCAGATCATGTTTGAGACCTTCAAC |
| | | | [9] CCCAGCCATGTACGTTGCTATCCAG |
| | | | [10] CGTTGCTATCCAGGCTGTGCTATCC |
| | | | [11] GGCTGTGCTATCCCTGTACGCCTCT |
| | | | [12] CGCCTCTGGCCGTACCACTGGCATC |
| | | | [13] TACCACTGGCATCGTGATGGACTCC |
| | | | [14] CGGTGACGGGGTCACCCACACTGTG |
| | | | [15] CCACACTGTGCCCATCTACGAGGGG |
| | | | [16] GCCCATCTACGAGGGGTATGCCCTC |
| | | | [17] TGCCATCCTGCGTCTGGACCTGGCT |
| | | | [18] TGATATCGCCGCGCTCGTCGTCGAC |
| | | | [19] CGTCGTCGACAACGGCTCCGGCATG |
| | | | [20] CGGCTCCGGCATGTGCAAGGCCGGC |
| 102-121 | M18728_at | 8 | [1] ACCCTCCTAATAGTCATACTAGTAG |
| | | | [2] CTAATAGTCATACTAGTAGTCATAC |
| | | | [3] GTCATACTAGTAGTCATACTCCCTG |
| | | | [4] CTAGTAGTCATACTCCCTGGTGTAG |
| | | | [5] ATGCAGCCAGCCATCAAATAGTGAA |
| | | | [6] TAGTGAATGGTCTCTCTTTGGCTGG |
| | | | [7] TAACCCATGAAGGATAAAAGCCCCA |
| | | | [8] ATAGCACTAATGCTTTAAGATTTGG |
| | | | [9] CTTTAAGATTTGGTCACACTCTCAC |
| | | | [10] GATTTGGTCACACTCTCACCTAGGT |
| | | | [11] CATTGAGCCAGTGGTGCTAAATGCT |
| | | | [12] GGTGCTAAATGCTACATACTCCAAC |
| | | | [13]TACATACTCCAACTGAAATGTTAAG |
| | | | [14] CTCCAACTGAAATGTTAAGGAAGAA |
| | | | [15] AACACAGGAGATTCCAGTCTACTTG |
| | | | [16] GCATAATACAGAAGTCCCCTCTACT |
| | | | [17] GTAACCTGAACTAATCTGATGTTAA |
| | | | [18] AATCTGATGTTAACCAATGTATTTA |
| | | | [19] CTGTTTCCTTGTTCCAATTTGACAA |
| | | | [20] GCTATCACTGTACTTGTAGAGTGGT |
| 122-141 | AFFX-HUMGAPDH/M33197_5_at | 7 | [1] GCGCCTGGTCACCAGGGCTGCTTTT |
| | | | [2] GGTCACCAGGGCTGCTTTTAACTCT |
| | | | [3] TGCTTTTAACTCTGGTAAAGTGGAT |
| | | | [4] GGATATTGTTGCCATCAATGACCCC |
| | | | [5] CATCAATGACCCCTTCATTGACCTC |
| | | | [6] CTTCATTGACCTCAACTACATGGTT |
| | | | [7] CAACTACATGGTTTACATGTTCCAA |
| | | | [8] GGTTTACATGTTCCAATATGATTCC |
| | | | [9] CCAATATGATTCCACCCATGGCAAA |
| | | | [10] TGATTCCACCCATGGCAAATTCCAT |
| | | | [11] ATTCCATGGCACCGTCAAGGCTGAG |
| | | | [12] TGGCACCGTCAAGGCTGAGAACGGG |
| | | | [13] CATCAATGGAAATCCCATCACCATC |
| | | | [14] TCCCATCACCATCTTCCAGGAGCGA |
| | | | [15] CTTCCAGGAGCGAGATCCCTCCAAA |
| | | | [16] GCGAGATCCCTCCAAAATCAAGTGG |
| | | | [17] CGATGCTGGCGCTGAGTACGTCGTG |
| | | | [18] CGTGGAGTCCACTGGCGTCTTCACC |
| | | | [19] CTTCACCACCATGGAGAAGGCTGGG |
| | | | [20] CGGATTTGGTCGTATTGGGCGCCTG |
| 142-161 | X00351_f_at | 6 | [1] TCCTCCTGAGCGCAAGTACTCCGTG |
| | | | [2] TGAGCGCAAGTACTCCGTGTGGATC |
| | | | [3] CTTCCAGCAGATGTGGATCAGCAAG |
| | | | [4] GTGGATCAGCAAGCAGGAGTATGAC |
| | | | [5] CCGCAAATGCTTCTAGGCGGACTAT |
| | | | [6] ATGCTTCTAGGCGGACTATGACTTA |
| | | | [7] TAACTTGCGCAGAAAACAAGATGAG |
| | | | [8] CAGCAGTCGGTTGGAGCGAGCATCC |
| | | | [9] CAATGTGGCCGAGGACTTTGATTGC |
| | | | [10] GGCCGAGGACTTTGATTGCACATTG |
| | | | [11] TGACGTGGACATCCGCAAAGACCTG |
| | | | [12] GTACGCCAACACAGTGCTGTCTGGC |
| | | | [13] CAACACAGTGCTGTCTGGCGGCACC |
| | | | [14] GTCTGGCGGCACCACCATGTACCCT |
| | | | [15] CACCATGTACCCTGGCATTGCCGAC |
| | | | [16] GTACCCTGGCATTGCCGACAGGATG |
| | | | [17] TGCCGACAGGATGCAGAAGGAGATC |
| | | | [18] GGAGATCACTGCCCTGGCACCCAGC |
| | | | [19] CCTGGCACCCAGCACAATGAAGATC |
| | | | [20] ACCCAGCACAATGAAGATCAAGATC |
| 162-181 | M77349_at | 5 | [1] TGAAGCACTACAGGAGGAATGCACC |
| | | | [2] AGCTCTCCGCCAATTTCTCTCAGAT |
| | | | [3] AATGTACATGGGCCGCACCATAATG |
| | | | [4] CATGGGCCGCACCATAATGAGATGT |
| | | | [5] CCGCACCATAATGAGATGTGAGCCT |
| | | | [6] TGGCTGTTAACCCACTGCATGCAGA |
| | | | [7] TTAACCCACTGCATGCAGAAACTTG |
| | | | [8] CACTGCATGCAGAAACTTGGATGTC |
| | | | [9] TGGAATTGACTGCCTATGCCAAGTC |
| | | | [10] TGACTGCCTATGCCAAGTCCCTGGA |
| | | | [11] CTCATAAAACATGAATCAAGCAATC |
| | | | [12] GAATCAAGCAATCCAGCCTCATGGG |
| | | | [13] TTGTAAAGCCCTTGCACAGCTGGAG |
| | | | [14] TGCACAGCTGGAGAAATGGCATCAT |
| | | | [15] GCATCATTATAAGCTATGAGTTGAA |
| | | | [16] AATGTTCTGTCAAATGTGTCTCACA |
| | | | [17] AATGTGTCTCACATCTACACGTGGC |
| | | | [18] TCTCACATCTACACGTGGCTTGGAG |
| | | | [19] TTCCCTATTGTGACAGAGCCATGGT |
| | | | [20] ATTGTGACAGAGCCATGGTGTGTTT |
| 182-192 | M34516_r_at | 3 | [1] TTCTCCCTGCACTCATGAAACCCCA |
| | | | [2] TCTCCCTGCACTCATGAAACCCCAA |
| | | | [3] GCACTCATGAAACCCCAATAAATAT |
| | | | [4] CACTCATGAAACCCCAATAAATATC |
| | | | [5] ACTCATGAAACCCCAATAAATATCC |
| | | | [6] CTCATGAAACCCCAATAAATATCCT |
| | | | [7] TCATGAAACCCCAATAAATATCCTC |
| | | | [8] CATGAAACCCCAATAAATATCCTCA |
| | | | [9] ATGAAACCCCAATAAATATCCTCAT |
| | | | [10] AAACCCCAATAAATATCCTCATTGA |
| | | | [11] AACCCCAATAAATATCCTCATTGAC |
| 193-199 | D49824_s_at | 2 | [1] GGCTGTCCTAGCAGTTGTGGTCATC |
| | | | [2] CTGTCCTAGCAGTTGTGGTCATCGG |
| | | | [3] TGTCCTAGCAGTTGTGGTCATCGGA |
| | | | [4] GTCCTAGCAGTTGTGGTCATCGGAG |
| | | | [5] TCCTAGCAGTTGTGGTCATCGGAGC |
| | | | [6] CTAGCAGTTGTGGTCATCGGAGCTG |
| | | | [7] TAGCAGTTGTGGTCATCGGAGCTGT |
| 220-239 | J03040 at | 1 | [1] GGTTTGCCTGAGGCTGTAACTGAGA |
| | | | [2] CCTGAGGCTGTAACTGAGAGAAAGA |
| | | | [3] ATTCTGGGGCTGTCTTATGAAAATA |
| | | | [4] ATAGACATTCTCACATAAGCCCAGT |
| | | | [5] ACATAAGCCCAGTTCATCACCATTT |
| | | | [6] TCACATTAGGCTGTTGGTTCAAACT |
| | | | [7] GAGCACGGACTGTCAGTTCTCTGGG |
| | | | [8] GGACTGTCAGTTCTCTGGGAAGTGG |
| | | | [9] GAAGTGGTCAGCGCATCCTGCAGGG |
| | | | [10] GTCAGCGCATCCTGCAGGGCTTCTC |
| | | | [11] TTTGGAGAACCAGGGCTCTTCTCAG |
| | | | [12] GAACCAGGGCTCTTCTCAGGGGCTC |
| | | | [13] TTCTCAGGGGCTCTAGGGACTGCCA |
| | | | [14] CTAGGGACTGCCAGGCTGTTTCAGC |
| | | | [15] TTTCAGCCAGGAAGGCCAAAATCAA |
| | | | [16] GGGATGGTCGGATCTCACAGGCTGA |
| | | | [17] GTCGGATCTCACAGGCTGAGAACTC |
| | | | [18] TCTCACAGGCTGAGAACTCGTTCAC |
| | | | [19] CCTCCAAGCATTTCATGAAAAAGCT |
| | | | [20] AGCATTTCATGAAAAAGCTGCTTCT |
| 240-259 | M13560_s_at | 1 | [1] CAGGATCTGGGCCCAGTCCCCATGT |
| | | | [2] GGCCCAGTCCCCATGTGAGAGCAGC |
| | | | [3] CCCATGTGAGAGCAGCAGAGGCGGT |
| | | | [4] AGAGCAGCAGAGGCGGTCTTCAACA |
| | | | [5] ACACAGCTACAGCTTTCTTGCTCCC |
| | | | [6] CAAGACAAACCAAGTCGGAACAGCA |
| | | | [7] CAAGTCGGAACAGCAGATAACAATG |
| | | | [8] TGCCCAATCTCCATCTGTCAACAGG |
| | | | [9] TGAGGTCCCAGGAAGTGGCCAAAAG |
| | | | [10] AGCTAGACAGATCCCCGTTCCTGAC |
| | | | [11] GACATCACAGCAGCCTCCAACACAA |
| | | | [12] CAACACAAGGCTCCAAGACCTAGGC |
| | | | [13] AAGACCTAGGCTCATGGACGAGATG |
| | | | [14] CCAGACCCCAGGCTGGACATGCTGA |
| | | | [15] CCTTTGGCCTTGGCTTTTCTAGCCT |
| | | | [16] TTGGCTTTTCTAGCCTATTTACCTG |
| | | | [17] AGCCTATTTACCTGCAGGCTGAGCC |
| | | | [18] GCTCAGCCAAGCTTGTTATCAGCTT |
| | | | [19] AAGCTTGTTATCAGCTTTCAGGGCC |
| | | | [20] ATCAGCTTTCAGGGCCATGGTTCAC |
| 260-264 | M34516_at | 1 | [1] TCCCTGCACTCATGAAACCCCAATA |
| | | | [2] CCCTGCACTCATGAAACCCCAATAA |
| | | | [3] CCTGCACTCATGAAACCCCAATAAA |
| | | | [4] CTGCACTCATGAAACCCCAATAAAT |
| | | | [5] TGCACTCATGAAACCCCAATAAATA |

The annotations of the selected mRNA probes are given in Table 13. The annotations were obtained from Bioconductor package hu6800.db [Marc Carlson, Seth Falcon, Herve Pages and Nianhua Li (2008). hu6800.db: Affymetrix HuGeneFL Genome Array annotation data (chip hu6800). R package version 2.2.3.] in combination with the information available via PubMed [http://www.ncbi.nlm.nih.gov/pubmed/].

**Table 13: Annotation of mRNA probes selected during LOO cross validation**

| **Seq-ID** | **Affymetrix ID** | **Accession number** | **RefSeq ID** | **Unigene ID** |
|---|---|---|---|---|
| 265 | AFFX-HSAC07/X00351_M_at | X00351 | NM_001101.2 | Hs.520640 |
| | | | | Hs.708120 |
| 266 | X03689_s_at | X03689 | NM_001402.2 | Hs.520703 |
| | | | | Hs.586423 |
| | | | | Hs.644639 |
| | | | | Hs.703481 |
| | | | | Hs.708256 |
| 265 | AFFX-HSAC07/X00351_5_at | X00351 | NM_001101.2 | Hs.520640 |
| | | | | Hs.708120 |
| 267 | M18728_at | M18728 | NM_002483.3 | Hs.466814 |
| 268 | AFFX-HUMGAPDH/M33197_5_at | M33197 | NM_002046.3 | Hs.544577 |
| | | | | Hs.592355 |
| | | | | Hs.711936 |
| 265 | X00351_f_at | X00351 | NM_001101.2 | Hs.520640 |
| | | | | Hs.708120 |
| 269 | M77349_at | M77349 | NM_000358.1 | Hs.369397 |
| | | | | Hs.645734 |
| 270 | M34516_r_at | M34516 | NM_001013618.1 | Hs.449585 |
| 271 | D49824_s_at | D49824 | NM_005514.5 | Hs.77961 |
| | | | | Hs.703277 |
| | | | | Hs.707171 |
| 272 | D00654_at | D00654 | NM_001615.3 | Hs.516105 |
| 273 | HG3044-HT3742_s_at | HG3044-HT3742 | NM_212482.1 | Hs.203717 |
| 274 | J03040_at | J03040 | NM_003118.2 | Hs.111779 |
| | | | | Hs.708558 |
| 275 | M13560_s_at | M13560 | NM_001025159.1 | Hs.436568 |
| 276 | M34516_at | M34516 | NM_020070.2 | Hs.348935 |

### Example 2.2: mRNA and microRNA: Kidney Cancer

We use the kidney cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001 ;98(26): 15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(7043):834-8] to develop a multilevel classifier using mRNA and microRNA data. The data are available from the home page of the Braoad Institute [see http://www.broad.mit.edu/publications/broad900 and http://www.broad.mit.edu/publications/broad993s]. Overall the mRNA and microRNA data of three normal tissues and four tumor tissues are available. The hybridisations were done with a bead-based array containing microRNA probes as well as with the Affymetrix HU6800 and HU35KsubA array for measuring the mRNA. We used only the mRNA data of the HU35KsubA arrays.

### Analysis:

For developing and validating a classifier based on these data we used single-hidden-layer neural networks [Ripley, B. D. (1996) Pattern Recognition and Neural Networks. Cambridge] in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.

The low level analysis (preprocessing) consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006). In each cross validation step we selected those six normalized microRNA probes, respectively those six normalized mRNA probes for classification which had the largest differences (in absolute value) of the mean values beyond those probes with p value equal or smaller than 0.1 by the Welch t-test. This is, we used a so called ranker for feature selection. Again there are numerous other feature selection strategies we could have used, some examples are given in Hall et al. (2003). Overall a microRNA, respectively mRNA probe may have been chosen up to seven times due to LOO cross-validation.

Using only microRNA data we obtain the estimated errors given in Table 14
Table 14

**Table 14: microRNA data, classification error via LOO cross validation**

| **classifier vs. true** | **kidney cancer** | **Normal** |
|---|---|---|
| **kidney cancer** | 50.0% | 66.7% |
| **normal** | 50.0% | 33.3% |

The estimated overall accuracy using LOO cross-validation is 42.9 %, sensitivity is 50%, specificity is 33.3%, positive predictive value is 50% and negative predictive value is 33.3%. In a second step we used the mRNA data of the HU35KsubA array. The results can be read off from Table 15. We get an estimated overall accuracy of 42.9 % again using LOO cross-validation. The estimated values for sensitivity, specificity, positive and negative predictive value are 50%, 33.3%, 50% and 33.3%, respectively.
Table 15

**Table 15: mRNA data, classification error via LOO cross validation**

| **classifier vs. true** | **kidney cancer** | **Normal** |
|---|---|---|
| **kidney cancer** | 50.0% | 66.7% |
| **normal** | 50.0% | 33.3% |

In the last step we combine microRNA and mRNA data and obtain the results given in Table 16. That is, the estimated overall accuracy using cross-validation is 71.4 %. Hence, this combination increases the overall accuracy from 42.9 % to 71.4 %. Sensitivity, specificity, positive and negative predictive value are increased to 75.0%, 66.7%, 75.0% and 66.7%, respectively.
Table 16

**Table 16: microRNA and mRNA data, classification error via LOO cross validation**

| **classifier vs. true** | **kidney cancer** | **Normal** |
|---|---|---|
| **kidney cancer** | 75.0% | 33.3% |
| **normal** | 25.0% | 66.7% |

The microRNA probes which were selected during cross-validation are given in Table 17.
Table 17

**Table 17: microRNA probes selected during LOO cross validation (1^{st} column is SEQ-ID-No)**

| **Seq-ID** | **Probe ID** | **Times selected** | **Probe sequence** |
|---|---|---|---|
| 277 | pre-control 3 | 5 + 5 + 5* | CTGACTGACTGACTGACTGACTG |
| 278 | pre-control 4 | 5 + 1 | TTGTACGTTTACATGGAGGTC |
| 279 | hsa-let-7b | 4 | AACCACACAACCTACTACCTCA |
| 280 | FVR506 | 4 + 1 | TGTATTCCTCGCCTGTCCAG |
| 281 | hsa-miR-320 | 2 | TCGCCCTCTCAACCCAGCTTTT |
| 282 | hsa-let-7a | 2 | AACTATACAACCTACTACCTCA |
| 283 | hsa-let-7c | 1 | AACCATACAACCTACTACCTCA |
| 284 | hsa-miR-30b | 1 | GCTGAGTGTAGGATGTTTACA |
| 285 | has-miR-10a | 1 | ACACAAATTCGGTTCTACAGGG |
| 286 | PTG20210 | 1 + 1 | CATTGAGGCTCGCTGAGAGT |
| 33 | hsa-miR-30e | 1 | TCCAGTCAAGGATGTTTACA |
| 287 | hsa-miR-339 | 1 | TGAGCTCCTGGAGGACAGGGA |
| 288 | pre-control 5 | 1 | CTTGTACCAGTTATCTGCAA |

| | | | |
|---|---|---|---|
| * *Some probes occur in replicates* | | | |

The results of the Sanger sequence search in accordance with Griffiths-Jones et al. 2008 for known human microRNAs are given in Table 18
Table 18

**Table 18: Results of the Sanger sequence search for known human microRNAs for microRNA probes selected during LOO cross validation (1^{st} column is SEQ-ID-No)**

| | | | |
|---|---|---|---|
| | pre-control 3 | | |
| 289 | pre-control 4 | hsa-mir-302d | CCUCUACUUUAACAUGGAGGCACUUGCUGUGACAU GACAAAAAUAAGUGCUUCCAUGUUUGAGUGUGG |
| 290 | hsa-let-7b | hsa-let-7b | CGGGGUGAGGUAGUAGGUUGUGUGGUUUCAGGGCA GUGAUGUUGCCCCUCGGAAGAUAACUAUACAACCUA CUGCCUUCCCUG |
| 291 | FVR506 | hsa-mir-1238 | GUGAGUGGAGCCCCAGUGUGUGGUUGGGGCCAU GGCGGGUGGGCAGCCCAGCCUCUGAGCCUUCCUCG UCUGUCUGCCCCAG |
| 292 | hsa-miR-320 | hsa-mir-320a | GCUUCGCUCCCUCCGCCUUCUCUUCCCGGUUCUU CCCGGAGUCGGGAAAAGCUGGGUUGAGAGGGCGAA AAAGGAUGAGGU |
| 293 | hsa-let-7a | hsa-let-7a | UGGGAUGAGGUAGUAGGUUGUAUAGUUUUAGGGUC ACACCCACCACUGGGAGAUAACUAUACAAUCUACUG UCUUUCCUA |
| 294 | hsa-let-7c | hsa-let-7c | GCAUCCGGGUUGAGGUAGUAGGUUGUAUGGUUUAG AGUUACACCCUGGGAGUUAACUGUACAACCUUCUAG CUUUCCUUGGAGC |
| 295 | hsa-miR-30b | hsa-mir-30b | ACCAAGUUUCAGUUCAUGUAAACAUCCUACACUCAG CUGUAAUACAUGGAUUGGCUGGGAGGUGGAUGUUU ACUUCAGCUGACUUGGA |
| | PTG20210 | | |
| 41 | hsa-miR-30e | hsa-mir-30e | GGGCAGUCUUUGCUACUGUAAACAUCCUUGACUGG AAGCUGUAAGGUGUUCAGAGGAGCUUUCAGUCGGA UGUUUACAGCGGCAGGCUGCCA |
| 21 | hsa-miR-339 | hsa-mir-339 | CGGGGCGGCCGCUCUCCCUGUCCUCCAGGAGCUCA CGUGUGCCUGCCUGUGAGCGCCUCGACGACAGAGC CGGCGCCUGCCCCAGUGUCUGCGC |
| 297 | pre-control 5 | hsa-mir-150 | CUCCCCAUGGCCCUGUCUCCCAACCCUUGUACCAG UGCUGGGCUCAGACCCUGGUACAGGCCUGGGGGAC AGGGACCUGGGGAC |

The mRNA probes which were selected during cross-validation are given in Table 19. The probe sequences were obtained from Bioconductor package hu35ksubaprobe (see The Bioconductor Project, www.bioconductor.org (2008). hu35ksubaprobe: Probe sequence data for microarrays of type hu35ksuba. R package version 2.2.0.). Table 19

**Table 19: mRNA probes selected during LOO cross validation**

| **Seq-ID** | **Affymetrix ID** | **Times selected** | **Probe sequence** | |
|---|---|---|---|---|
| 298-313 | AA285290_at | 5 | [1] | GGAAAGCGCCGAGATGACGGGCTTT |
| | | | [2] | GATGACGGGCTTTCTGCTGCCGCCC |
| | | | [3] | CCCAAGTAGCTTTGTGGCTTCGTGT |
| | | | [4] | TAGCTTTGTGGCTTCGTGTCCAACC |
| | | | [5] | TGTGGCTTCGTGTCCAACCCTCTTG |
| | | | [6] | CGCCTGTGTGCCTGGAGCCAGTCCC |
| | | | [7] | GCTCGCGTTTCCTCCTGTAGTGCTC |
| | | | [8] | GTTTCCTCCTGTAGTGCTCACAGGT |
| | | | [9] | AGTGCTCACAGGTCCCAGCACCGAT |
| | | | [10] | TCCCAGCACCGATGGCATTCCCTTT |
| | | | [11] | TCCCTTTGCCCTGAGTCTGCAGCGG |
| | | | [12] | TGCCCTGAGTCTGCAGCGGGTCCCT |
| | | | [13] | TCAGGTAGCCTCTCTTCCCCTTGGG |
| | | | [14] | ACCCGCGGTAACCAGCGTGAGCTCG |
| | | | [15] | GCCCGCCAGAAGAATATGAAAAAGC |
| | | | [16] | GACTCGGTTAAGGGAAAGCGCCGAG |
| 314-328 | AA464334_s_at | 4 | [1] | TTATGAATGTCCAAATCTGTGTTTC |
| | | | [2] | ATGAATGTCCAAATCTGTGTTTCCC |
| | | | [3] | GAATGTCCAAATCTGTGTTTCCCCC |
| | | | [4] | ATGTCCAAATCTGTGTTTCCCCCTG |
| | | | [5] | CTCCCAGACTGTGTGGCCAGTTGAA |
| | | | [6] | AGACTGTGTGGCCAGTTGAAAGTGT |
| | | | [7] | ACTGTGTGGCCAGTTGAAAGTGTCT |
| | | | [8] | TGGCCAGTTGAAAGTGTCTGGTTTG |
| | | | [9] | TTGAAAGTGTCTGGTTTGTGTTCAT |
| | | | [10] | AGTGTCTGGTTTGTGTTCATCTCTC |
| | | | [11] | TGTCTGGTTTGTGTTCATCTCTCCC |
| | | | [12] | GTGTTCATCTCTCCCTCATTTCTGG |
| | | | [13] | TGCATCCACGCCTCTTTTGGACATT |
| | | | [14] | CATCCACGCCTCTTTTGGACATTAA |
| | | | [15] | TCCACGCCTCTTTTGGACATTAAAG |
| 329-343 | AA397610_at | 3 | [1] | GGTGGCCTTCTTGCAGGTCCCCGTA |
| | | | [2] | TGGCCTTCTTGCAGGTCCCCGTAGC |
| | | | [3] | GGCCTTCTTGCAGGTCCCCGTAGCA |
| | | | [4] | GCCTTCTTGCAGGTCCCCGTAGCAC |
| | | | [5] | TCTTGCAGGTCCCCGTAGCACCCTG |
| | | | [6] | TGCAGGTCCCCGTAGCACCCTGAGC |
| | | | [7] | AGGTCCCCGTAGCACCCTGAGCCTG |
| | | | [8] | GGTCCCCGTAGCACCCTGAGCCTGT |
| | | | [9] | CCGTAGCACCCTGAGCCTGTACCTT |
| | | | [10] | TAGCACCCTGAGCCTGTACCTTGGG |
| | | | [11] | CACCCTGAGCCTGTACCTTGGGTGG |
| | | | [12] | ACCCTGAGCCTGTACCTTGGGTGGC |
| | | | [13] | CCCTGAGCCTGTACCTTGGGTGGCA |
| | | | [14] | GAGCCTGTACCTTGGGTGGCACTTG |
| | | | [15] | GCCTGTACCTTGGGTGGCACTTGTT |
| 344-359 | RC_AA292427_s_at | 3 | [1] | TGCTGCCTCTGGGGACATGCGGAGT |
| | | | [2] | GGGGAAGCCTTCCTCTCAATTTGTT |
| | | | [3] | GGGAAGCCTTCCTCTCAATTTGTTG |
| | | | [4] | GGAAGCCTTCCTCTCAATTTGTTGT |
| | | | [5] | GAAGCCTTCCTCTCAATTTGTTGTC |
| | | | [6] | AAGCCTTCCTCTCAATTTGTTGTCA |
| | | | [7] | AGCCTTCCTCTCAATTTGTTGTCAG |
| | | | [8] | CCTTCCTCTCAATTTGTTGTCAGTG |
| | | | [9] | CTTCCTCTCAATTTGTTGTCAGTGA |
| | | | [10] | TTCCTCTCAATTTGTTGTCAGTGAA |
| | | | [11] | TCCTCTCAATTTGTTGTCAGTGAAA |
| | | | [12] | CCTCTCAATTTGTTGTCAGTGAAAT |
| | | | [13] | CTCTCAATTTGTTGTCAGTGAAATT |
| | | | [14] | AATTCCAATAAATGGGATTTGCTCT |
| | | | [15] | TGAGGGTGCACGTCTTCCCTCCTGT |
| | | | [16] | TGGAGTGCTGCCTCTGGGGACATGC |
| 360-374 | RC_AA465694_r_at | 3 | [1] | GGTTAATCCGCAAGCCCCAGCCCCG |
| | | | [2] | TTAATCCGCAAGCCCCAGCCCCGAG |
| | | | [3] | GGCGTCCCCCAGAGCCTGAGAAAGC |
| | | | [4] | CCCCAGAGCCTGAGAAAGCGCCTCC |
| | | | [5] | CCAGAGCCTGAGAAAGCGCCTCCCG |
| | | | [6] | GAGCCTGAGAAAGCGCCTCCCGCTG |
| | | | [7] | GCCTGAGAAAGCGCCTCCCGCTGCC |
| | | | [8] | CTGAGAAAGCGCCTCCCGCTGCCCC |
| | | | [9] | TGCCCCGACGCGGCCCTCGGCCCTG |
| | | | [10] | CTCGGCCCTGGAGCTGAAGGTGGAG |
| | | | [11] | CGGCCCTGGAGCTGAAGGTGGAGGA |
| | | | [12] | GCCCTGGAGCTGAAGGTGGAGGAGC |
| | | | [13] | CCTGGAGCTGAAGGTGGAGGAGCTG |
| | | | [14] | GCTGAAGGTGGAGGAGCTGGAGGAG |
| | | | [15] | AAGGTGGAGGAGCTGGAGGAGAAGG |
| 375-390 | AA422123_f_at | 2 | [1] | GACTGCTTGAAACCAGGAGTTTGAG |
| | | | [2] | GCTTGAAACCAGGAGTTTGAGACCA |
| | | | [3] | AACCAGGAGTTTGAGACCAGCCTGA |
| | | | [4] | TTGAGACCAGCCTGAGCAACAAAGC |
| | | | [5] | AGACCAGCCTGAGCAACAAAGCAAG |
| | | | [6] | GAGCAACAAAGCAAGACCCCATCTC |
| | | | [7] | CAACAAAGCAAGACCCCATCTCTAT |
| | | | [8] | AAGCAAGACCCCATCTCTATAAAAA |
| | | | [9] | AAGACAGGGTCTTGCTCATGTTGTA |
| | | | [10] | ATTAGTTGGGCATGGTGGCACATGC |
| | | | [11] | AGTTGGGCATGGTGGCACATGCCTG |
| | | | [12] | ATCATCTGAGCCTCAGGAGGTTGAG |
| | | | [13] | ATCTGAGCCTCAGGAGGTTGAGGCT |
| | | | [14] | TGAGGCTGCAGTGAGCTGTGACTGC |
| | | | [15] | CTTGCTCATGTTGTACATTCATCAT |
| | | | [16] | AAGAGGCTGGGTGCAGTGGCTCACA |
| 391-410 | AFFX-HUMGAPDH/M33197_3 _at | 2 | [1] | TCATTTCCTGGTATGACAACGAATT |
| | | | [2] | ACAACGAATTTGGCTACAGCAACAG |
| | | | [3] | GGGTGGTGGACCTCATGGCCCACAT |
| | | | [4] | TCATGGCCCACATGGCCTCCAAGGA |
| | | | [5] | ACATGGCCTCCAAGGAGTAAGACCC |
| | | | [6] | AGGAGTAAGACCCCTGGACCACCAG |
| | | | [7] | GCCCCAGCAAGAGCACAAGAGGAAG |
| | | | [8] | GAGAGAGACCCTCACTGCTGGGGAG |
| | | | [9] | CCTCACTGCTGGGGAGTCCCTGCCA |
| | | | [10] | CCTCCTCACAGTTGCCATGTAGACC |
| | | | [11] | AGTTGCCATGTAGACCCCTTGAAGA |
| | | | [12] | CATGTAGACCCCTTGAAGAGGGGAG |
| | | | [13] | TAGGGAGCCGCACCTTGTCATGTAC |
| | | | [14] | GCCGCACCTTGTCATGTACCATCAA |
| | | | [15] | TGTCATGTACCATCAATAAAGTACC |
| | | | [16] | CCTCTGACTTCAACAGCGACACCCA |
| | | | [17] | GGGCTGGCATTGCCCTCAACGACCA |
| | | | [18] | CCCTCAACGACCACTTTGTCAAGCT |
| | | | [19] | ACCACTTTGTCAAGCTCATTTCCTG |
| | | | [20] | TTGTCAAGCTCATTTCCTGGTATGA |
| 411-426 | RC_AA130645_s_at | 2 | [1] | GAATTCTGGTACCGTCAGCATCCAC |
| | | | [2] | GAGAGAGACCTCATCTTTCATGCTT |
| | | | [3] | TGACTCTCCTGGGGGCACCTCCTAT |
| | | | [4] | ACTCTCCTGGGGGCACCTCCTATGA |
| | | | [5] | TCCTGGGGGCACCTCCTATGAGAGA |
| | | | [6] | CCTGGGGGCACCTCCTATGAGAGAT |
| | | | [7] | CTGGGGGCACCTCCTATGAGAGATA |
| | | | [8] | TGGGGGCACCTCCTATGAGAGATAC |
| | | | [9] | GGGGGCACCTCCTATGAGAGATACG |
| | | | [10] | GGGGCACCTCCTATGAGAGATACGA |
| | | | [11] | GGGCACCTCCTATGAGAGATACGAT |
| | | | [12] | GGCACCTCCTATGAGAGATACGATT |
| | | | [13] | GCACCTCCTATGAGAGATACGATTG |
| | | | [14] | CACCTCCTATGAGAGATACGATTGC |
| | | | [15] | ACCTCCTATGAGAGATACGATTGCT |
| | | | [16] | CCTCCTATGAGAGATACGATTGCTA |
| 427-442 | RC_AA236365_s_at | 2 | [1] | CTCCTATTCCGGACTCAGACCTCTG |
| | | | [2] | TCCTATTCCGGACTCAGACCTCTGA |
| | | | [3] | CCTATTCCGGACTCAGACCTCTGAC |
| | | | [4] | CTATTCCGGACTCAGACCTCTGACC |
| | | | [5] | ATTCCGGACTCAGACCTCTGACCCT |
| | | | [6] | TTCCGGACTCAGACCTCTGACCCTG |
| | | | [7] | CGGACTCAGACCTCTGACCCTGCAA |
| | | | [8] | GGACTCAGACCTCTGACCCTGCAAT |
| | | | [9] | ACTCAGACCTCTGACCCTGCAATGC |
| | | | [10] | CAGACCTCTGACCCTGCAATGCTGC |
| | | | [11] | ACCTCTGACCCTGCAATGCTGCCTA |
| | | | [12] | TCTGACCCTGCAATGCTGCCTACCA |
| | | | [13] | CTGACCCTGCAATGCTGCCTACCAT |
| | | | [14] | TGACCCTGCAATGCTGCCTACCATG |
| | | | [15] | ACCCTGCAATGCTGCCTACCATGAT |
| | | | [16] | CCTGCAATGCTGCCTACCATGATTG |
| 443-458 | RC_AA304344_f_at | 2 | [1] | AGGCACGTACCACCATGCCCAGATA |
| | | | [2] | TTTTTGAGACAAAGTCCTCACTCT |
| | | | [3] | GGGGTTTCACCATGTTGGCTAGGAT |
| | | | [4] | CCATGTTGGCTAGGATGGTCTCCAT |
| | | | [5] | GTTGGCTAGGATGGTCTCCATCGCC |
| | | | [6] | CTAGGATGGTCTCCATCGCCTGACC |
| | | | [7] | TGAGACAAAGTCCTCACTCTGTCAC |
| | | | [8] | CTTGGCCTCCCAAAGTGCTGGGATT |
| | | | [9] | CCTCCCAAAGTGCTGGGATTACAGG |
| | | | [10] | GGATTACAGGCATGAGCCACCACAG |
| | | | [11] | CAAAGTCCTCACTCTGTCACCAAGT |
| | | | [12] | GCATGAGCCACCACAGCTGGCCGTA |
| | | | [13] | GAGCCACCACAGCTGGCCGTAAATA |
| | | | [14] | GTGCAGTGGCAGCAATCTCAGCTCA |
| | | | [15] | GTGGCAGCAATCTCAGCTCACTGCA |
| | | | [16] | AGCAATCTCAGCTCACTGCAAACCT |
| 459-473 | T89571_f_at | 2 | [1] | CACCGCGCCTGGCCCTAAATAGATT |
| | | | [2] | GGGATTCATCATGTTGACCAGGCTG |
| | | | [3] | TTCATCATGTTGACCAGGCTGGCCT |
| | | | [4] | TGTTTGTCTTTCTGATAGGTTGAAA |
| | | | [5] | TGTCTTTCTGATAGGTTGAAAATTG |
| | | | [6] | GTTGACCAGGCTGGCCTCAAACTCC |
| | | | [7] | ACCAGGCTGGCCTCAAACTCCTGAC |
| | | | [8] | AGGCTGGCCTCAAACTCCTGACTTC |
| | | | [9] | TGGCCTCAAACTCCTGACTTCAAGC |
| | | | [10] | CTCAAACTCCTGACTTCAAGCGATC |
| | | | [11] | AAACTCCTGACTTCAAGCGATCTCC |
| | | | [12] | TTGGCCTCCCAAAGTGCTGGGATTG |
| | | | [13] | CCTCCCAAAGTGCTGGGATTGCAGG |
| | | | [14] | GCTGGGATTGCAGGTGTGAGCCACC |
| | | | [15] | ATTGCAGGTGTGAGCCACCGCGCCT |
| 474-493 | AFFX-HSAC07/X00351_3_at | 1 | [1] | TCTTGACAAAACCTAACTTGCGCAG |
| | | | [2] | ATGAGATTGGCATGGCTTTATTTGT |
| | | | [3] | GCAGTCGGTTGGAGCGAGCATCCCC |
| | | | [4] | CCAAAGTTCACAATGTGGCCGAGGA |
| | | | [5] | AAGTTCACAATGTGGCCGAGGACTT |
| | | | [6] | ATGTGGCCGAGGACTTTGATTGCAC |
| | | | [7] | CCGAGGACTTTGATTGCACATTGTT |
| | | | [8] | TTTAATAGTCATTCCAAATATGAGA |
| | | | [9] | AGTCATTCCAAATATGAGATGCATT |
| | | | [10] | TGTTACAGGAAGTCCCTTGCCATCC |
| | | | [11] | TACAGGAAGTCCCTTGCCATCCTAA |
| | | | [12] | TCCCTTGCCATCCTAAAAGCCACCC |
| | | | [13] | CTTCTCTCTAAGGAGAATGGCCCAG |
| | | | [14] | GAGGTGATAGCATTGCTTTCGTGTA |
| | | | [15] | TATTTTGAATGATGAGCCTTCGTGC |
| | | | [16] | TTTGAATGATGAGCCTTCGTGCCCC |
| | | | [17] | GTATGAAGGCTTTTGGTCTCCCTGG |
| | | | [18] | GGTGGAGGCAGCCAGGGCTTACCTG |
| | | | [19] | CAGGGCTTACCTGTACACTGACTTG |
| | | | [20] | TTACCTGTACACTGACTTGAGACCA |
| 494-562 | hum_alu_at | 1 | [1] | GCCTGGCCAACATGGTGAAACCCCG |
| | | | [2] | GCGCGCGCCTGTAATCCCAGCTACT |
| | | | [3] | GCGCGCCTGTAATCCCAGCTACTCG |
| | | | [4] | CGCGCCTGTAATCCCAGCTACTCGG |
| | | | [5] | GCGCCTGTAATCCCAGCTACTCGGG |
| | | | [6] | CGCCTGTAATCCCAGCTACTCGGGA |
| | | | [7] | GCCTGTAATCCCAGCTACTCGGGAG |
| | | | [8] | CCTGTAATCCCAGCTACTCGGGAGG |
| | | | [9] | CTGTAATCCCAGCTACTCGGGAGGC |
| | | | [10] | TGTAATCCCAGCTACTCGGGAGGCT |
| | | | [11] | GTAATCCCAGCTACTCGGGAGGCTG |
| | | | [12] | TAATCCCAGCTACTCGGGAGGCTGA |
| | | | [13] | AATCCCAGCTACTCGGGAGGCTGAG |
| | | | [14] | ATCCCAGCTACTCGGGAGGCTGAGG |
| | | | [15] | TCCCAGCTACTCGGGAGGCTGAGGC |
| | | | [16] | CCCAGCTACTCGGGAGGCTGAGGCA |
| | | | [17] | CCAGCTACTCGGGAGGCTGAGGCAG |
| | | | [18] | TGGTGGCTCACGCCTGTAATCCCAG |
| | | | [19] | GAGCCGAGATCGCGCCACTGCACTC |
| | | | [20] | GTGGCTCACGCCTGTAATCCCAGCA |
| | | | [21] | CACTGCACTCCAGCCTGGGCGACAG |
| | | | [22] | ACTGCACTCCAGCCTGGGCGACAGA |
| | | | [23] | CTGCACTCCAGCCTGGGCGACAGAG |
| | | | [24] | TGCACTCCAGCCTGGGCGACAGAGC |
| | | | [25] | GCACTCCAGCCTGGGCGACAGAGCG |
| | | | [26] | CACTCCAGCCTGGGCGACAGAGCGA |
| | | | [27] | TGGCTCACGCCTGTAATCCCAGCAC |
| | | | [28] | ACTCCAGCCTGGGCGACAGAGCGAG |
| | | | [29] | CTCCAGCCTGGGCGACAGAGCGAGA |
| | | | [30] | TCCAGCCTGGGCGACAGAGCGAGAC |
| | | | [31] | CCAGCCTGGGCGACAGAGCGAGACT |
| | | | [32] | CAGCCTGGGCGACAGAGCGAGACTC |
| | | | [33] | AGCCTGGGCGACAGP,GCGAGACTCC |
| | | | [34] | GGCTCACGCCTGTAATCCCAGCACT |
| | | | [35] | GCTCACGCCTGTAATCCCAGCACTT |
| | | | [36] | CTCACGCCTGTAATCCCAGCACTTT |
| | | | [37] | TCACGCCTGTAATCCCAGCACTTTG |
| | | | [38] | CACGCCTGTAATCCCAGCACTTTGG |
| | | | [39] | ACGCCTGTAATCCCAGCACTTTGGG |
| | | | [40] | CGCCTGTAATCCCAGCACTTTGGGA |
| | | | [41] | GCCTGTAATCCCAGCACTTTGGGAG |
| | | | [42] | CCTGTAATCCCAGCACTTTGGGAGG |
| | | | [43] | CTGTAATCCCAGCACTTTGGGAGGC |
| | | | [44] | TGTAATCCCAGCACTTTGGGAGGCC |
| | | | [45] | GTAATCCCAGCACTTTGGGAGGCCG |
| | | | [46] | TAATCCCAGCACTTTGGGAGGCCGA |
| | | | [47] | AATCCCAGCACTTTGGGAGGCCGAG |
| | | | [48] | ATCCCAGCACTTTGGGAGGCCGAGG |
| | | | [49] | TCCCAGCACTTTGGGAGGCCGAGGT |
| | | | [50] | CCCAGCACTTTGGGAGGCCGAGGTG |
| | | | [51] | GTGGATCACCTGAGGTCAGGAGTTC |
| | | | [52] | GGATCACCTGAGGTCAGGAGTTCAA |
| | | | [53] | GATCACCTGAGGTCAGGAGTTCAAG |
| | | | [54] | ATCACCTGAGGTCAGGAGTTCAAGA |
| | | | [55] | TCACCTGAGGTCAGGAGTTCAAGAC |
| | | | [56] | AGGAGTTCAAGACCAGCCTGGCCAA |
| | | | [57] | GGAGTTCAAGACCAGCCTGGCCAAC |
| | | | [58] | GAGTTCAAGACCAGCCTGGCCAACA |
| | | | [59] | AGTTCAAGACCAGCCTGGCCAACAT |
| | | | [60] | GTTCAAGACCAGCCTGGCCAACATG |
| | | | [61] | TTCAAGACCAGCCTGGCCAACATGG |
| | | | [62] | TCAAGACCAGCCTGGCCAACATGGT |
| | | | [63] | CAAGACCAGCCTGGCCAACATGGTG |
| | | | [64] | AAGACCAGCCTGGCCAACATGGTGA |
| | | | [65] | AGACCAGCCTGGCCAACATGGTGAA |
| | | | [66] | GACCAGCCTGGCCAACATGGTGAAA |
| | | | [67] | ACCAGCCTGGCCAACATGGTGAAAC |
| | | | [68] | CCAGCCTGGCCAACATGGTGAAACC |
| | | | [69] | CAGCCTGGCCAACATGGTGAAACCC |
| 563-578 | R69648_at | 1 | [1] | TAGAATTCTGTGCAGATGTCCTGAC |
| | | | [2] | AATTCTGTGCAGATGTCCTGACTTG |
| | | | [3] | TGACTTGGCAATTTTGTGTCCCTGC |
| | | | [4] | GGCAATTTTGTGTCCCTGCCTCACT |
| | | | [5] | GTCCTAGTGTTGTTCTGCCTCCTGT |
| | | | [6] | TTGTTCTGCCTCCTGTCCTCTCTTG |
| | | | [7] | CTGTCCTCTCTTGCTCTCTTGTCAG |
| | | | [8] | GCTCTCTTGTCAGTCTCTGGCTTCC |
| | | | [9] | GTCTCTGGCTTCCTCGGCCCCATTT |
| | | | [10] | GGCCCCATTTCACTTCACTGAGTCC |
| | | | [11] | CCCATTTCACTTCACTGAGTCCTGA |
| | | | [12] | TCACTTCACTGAGTCCTGACACCCA |
| | | | [13] | AAGGGTCTGTTCTGCTCAGCTCCAT |
| | | | [14] | TGCTCAGCTCCATGTCCCCCATTTT |
| | | | [15] | TTTACAGCATCCTGCACTCCAGCCT |
| | | | [16] | TCCTCCACAATAAAACTGGGGACTG |
| 579-593 | RC_AA232686_s_at | 1 | [1] | GCTGAGGCTCCCTTGCCTGACTGTG |
| | | | [2] | GAGGCTCCCTTGCCTGACTGTGACT |
| | | | [3] | GGCTCCCTTGCCTGACTGTGACTTG |
| | | | [4] | GCTCCCTTGCCTGACTGTGACTTGT |
| | | | [5] | CTCCCTTGCCTGACTGTGACTTGTG |
| | | | [6] | TCCCTTGCCTGACTGTGACTTGTGC |
| | | | [7] | CCCTTGCCTGACTGTGACTTGTGCC |
| | | | [8] | CCTTGCCTGACTGTGACTTGTGCCT |
| | | | [9] | CTTGCCTGACTGTGACTTGTGCCTC |
| | | | [10] | CTGACTGTGACTTGTGCCTCTCTCC |
| | | | [11] | TGACTGTGACTTGTGCCTCTCTCCT |
| | | | [12] | GACTGTGACTTGTGCCTCTCTCCTG |
| | | | [13] | CTGTGACTTGTGCCTCTCTCCTGCC |
| | | | [14] | GGTGGGCAGGTGACCCAAGGAACCT |
| | | | [15] | CAGGTGACCCAAGGAACCTTTCTGG |
| 594-609 | RC_AA417588_at | 1 | [1] | TGAAGGTACTGAACGCCACCTCACT |
| | | | [2] | AGGTACTGAACGCCACCTCACTGTA |
| | | | [3] | GTACTGAACGCCACCTCACTGTAAG |
| | | | [4] | TGAACGCCACCTCACTGTAAGACGG |
| | | | [5] | AACGCCACCTCACTGTAAGACGGTA |
| | | | [6] | ACGCCACCTCACTGTAAGACGGTAG |
| | | | [7] | GCCACCTCACTGTAAGACGGTAGAT |
| | | | [8] | CCACCTCACTGTAAGACGGTAGATT |
| | | | [9] | ACCTCACTGTAAGACGGTAGATTTT |
| | | | [10] | CCTCACTGTAAGACGGTAGATTTTG |
| | | | [11] | TCACTGTAAGACGGTAGATTTTGTA |
| | | | [12] | GACAGGGCTGCCTTCTGGGTGATGA |
| | | | [13] | ACAGGGCTGCCTTCTGGGTGATGAG |
| | | | [14] | AGGGCTGCCTTCTGGGTGATGAGAA |
| | | | [15] | AATCAGATGGGATGGCTGCACGGCG |
| | | | [16] | CTGCACGGCGTGGTGAAGGTACTGA |
| 610-624 | RC_AA459310_r_at | 1 | [1] | CTGCAGTTCATGTCCCCCGCCAGGC |
| | | | [2] | CCCCGCCAGGCCTCGAGGCTCAGGG |
| | | | [3] | CGCCAGGCCTCGAGGCTCAGGGTGG |
| | | | [4] | GCCTCGAGGCTCAGGGTGGGAGAGG |
| | | | [5] | GAGGCTCAGGGTGGGAGAGGGCCCC |
| | | | [6] | GCTCAGGGTGGGAGAGGGCCCCGGG |
| | | | [7] | CCCCGGGCTGCCCTGTCACTCCTCT |
| | | | [8] | CGGGCTGCCCTGTCACTCCTCTAAC |
| | | | [9] | GCTGCCCTGTCACTCCTCTAACACT |
| | | | [10] | CCTGTCACTCCTCTAACACTTCCCT |
| | | | [11] | TCACTCCTCTAACACTTCCCTCCCG |
| | | | [12] | CTCCTCTAACACTTCCCTCCCGTGT |
| | | | [13] | CCCCAACATGCCCTGTAATAAAATT |
| | | | [14] | CAACATGCCCTGTAATAAAATTAGA |
| | | | [15] | CATGCCCTGTAATAAAATTAGAGAA |
| 625-639 | RC_AA496904_at | 1 | [1] | TAGAATGACCCTTGGGAACAGTGAA |
| | | | [2] | GACCCTTGGGAACAGTGAACGTAGA |
| | | | [3] | TTTAGCAGAGTTTGTGACCAAAGTC |
| | | | [4] | GCTCTGGCTGCCTTCTGCATTTATT |
| | | | [5] | GCTGCCTTCTGCATTTATTTGCCTT |
| | | | [6] | GCCTTGGCCTGTTGTCTTCCCCTAT |
| | | | [7] | GCCTGTTGTCTTCCCCTATTTTCTG |
| | | | [8] | TGTCTTCCCCTATTTTCTGTCCCAG |
| | | | [9] | CTATTTTCTGTCCCAGCTCATCCGT |
| | | | [10] | TTTTCTGTCCCAGCTCATCCGTGTC |
| | | | [11] | TCTGTCCCAGCTCATCCGTGTCTCT |
| | | | [12] | GTCCCAGCTCATCCGTGTCTCTGAA |
| | | | [13] | CCAGCTCATCCGTGTCTCTGAAGAA |
| | | | [14] | GCTCATCCGTGTCTCTGAAGAACAA |
| | | | [15] | CCGTGTCTCTGAAGAACAAATATGC |
| 640-654 | RC_D59847_at | 1 | [1] | TTGCCACCCTGAGCATGCCCGGAT |
| | | | [2] | GGATCCCGTGCACCCTGGGACCCAG |
| | | | [3] | TCCCGTGCACCCTGGGACCCAGAAG |
| | | | [4] | CGTGCACCCTGGGACCCAGAAGTGC |
| | | | [5] | CCGCCAGCACGTCCAGAGCAACTTA |
| | | | [6] | GCCAGCACGTCCAGAGCAACTTACC |
| | | | [7] | AGCACGTCCAGAGCAACTTACCCCG |
| | | | [8] | GCACGTCCAGAGCAACTTACCCCGG |
| | | | [9] | CCGTGCCGCCGACCACGATGTGGGC |
| | | | [10] | CGTGCCGCCGACCACGATGTGGGCT |
| | | | [11] | TGCCGCCGACCACGATGTGGGCTCT |
| | | | [12] | CGCCGACCACGATGTGGGCTCTGAG |
| | | | [13] | GACCACGATGTGGGCTCTGAGCTGC |
| | | | [14] | CACGATGTGGGCTCTGAGCTGCCCC |
| | | | [15] | TGTGAAACGCCTAGAGACCCCGGCG |
| 655-669 | RC_D60607_at | 1 | [1] | TCACAGCCCCGTTCAGCTGGTGGCT |
| | | | [2] | CCCCGTTCAGCTGGTGGCTTTTAGA |
| | | | [3] | TTTTAGAGGCTTCCAGAGTGTGCTT |
| | | | [4] | CCAGAGTGTGCTTGGCCCCTTTACC |
| | | | [5] | TGGCCCCTTTACCTCTATGCCATTG |
| | | | [6] | CTCTATGCCATTGGGCCCAGGGGGA |
| | | | [7] | CCTTTCTGTGTCTTGCTTGCCCCGT |
| | | | [8] | TGTGTCTTGCTTGCCCCGTGTCTCC |
| | | | [9] | TTGCTTGCCCCGTGTCTCCCAGTGA |
| | | | [10] | GCCCCGTGTCTCCCAGTGAGTGGCC |
| | | | [11] | TGTCTCCCAGTGAGTGGCCGCCCTG |
| | | | [12] | CGGACAAGTCGCAGCCTCAGGGGGA |
| | | | [13] | AGTCGCAGCCTCAGGGGGACCTCCC |
| | | | [14] | CTGGCACTGCATCTTTCTGGGCCTG |
| | | | [15] | CTTTCTGGGCCTGGCTCTGCTGCCT |
| 670-684 | T30851_i_at | 1 | [1] | CAGAGTTATAAGCCCCAAACAGGTC |
| | | | [2] | AGAGTTATAAGCCCCAAACAGGTCA |
| | | | [3] | GAGTTATAAGCCCCAAACAGGTCAT |
| | | | [4] | AGTTATAAGCCCCAAACAGGTCATG |
| | | | [5] | GTTATAAGCCCCAAACAGGTCATGC |
| | | | [6] | TTATAAGCCCCAAACAGGTCATGCT |
| | | | [7] | TATAAGCCCCAAACAGGTCATGCTC |
| | | | [8] | ATAAGCCCCAAACAGGTCATGCTCC |
| | | | [9] | TAAGCCCCAAACAGGTCATGCTCCA |
| | | | [10] | AAGCCCCAAACAGGTCATGCTCCAA |
| | | | [11] | AGCCCCAAACAGGTCATGCTCCAAT |
| | | | [12] | GCCCCAAACAGGTCATGCTCCAATA |
| | | | [13] | CCCCAAACAGGTCATGCTCCAATAA |
| | | | [14] | CCCAAACAGGTCATGCTCCAATAAA |
| | | | [15] | CCAAACAGGTCATGCTCCAATAAAA |
| 685-700 | T80746_s_at | 1 | [1] | CTTGCAACCTCCGGGACCATCTTCT |
| | | | [2] | GCAACCTCCGGGACCATCTTCTCGG |
| | | | [3] | GCTTCTGGGACCTGCCAGCACCGTT |
| | | | [4] | GGGACCTGCCAGCACCGTTTTTGTG |
| | | | [5] | TGCCAGCACCGTTTTTGTGGTTAGC |
| | | | [6] | CAGCACCGTTTTTGTGGTTAGCTCC |
| | | | [7] | TTGCCAACCAACCATGAGCTCCCAG |
| | | | [8] | GCCAACCAACCATGAGCTCCCAGAT |
| | | | [9] | AACCAACCATGAGCTCCCAGATTCG |
| | | | [10] | CCATGAGCTCCCAGATTCGTCAGAA |
| | | | [11] | TGAGCTCCCAGATTCGTCAGAATTA |
| | | | [12] | GCTCCCAGATTCGTCAGAATTATTC |
| | | | [13] | CCCAGATTCGTCAGAATTATTCCAC |
| | | | [14] | GATTCGTCAGAATTATTCCACCGAC |
| | | | [15] | TCGTCAGAATTATTCCACCGACGTG |
| | | | [16] | TCAGAATTATTCCACCGACGTGGAG |
| 701-716 | X01677_s_at | 1 | [1] | ACTGGCATGGCCTTCCGTGTCCCCA |
| | | | [2] | CCACTGCCAACGTGTCAGTGGTGGA |
| | | | [3] | ACTGCCAACGTGTCAGTGGTGGACC |
| | | | [4] | TGCCAACGTGTCAGTGGTGGACCTG |
| | | | [5] | CCAACGTGTCAGTGGTGGACCTGAC |
| | | | [6] | CGTGTCAGTGGTGGACCTGACCTGC |
| | | | [7] | GTCAGTGGTGGACCTGACCTGCCGT |
| | | | [8] | CAGTGGTGGACCTGACCTGCCGTCT |
| | | | [9] | GTGGTGGACCTGACCTGCCGTCTAG |
| | | | [10] | GGTGGACCTGACCTGCCGTCTAGAA |
| | | | [11] | GACCTGACCTGCCGTCTAGAAAAAC |
| | | | [12] | CTGACCTGCCGTCTAGAAAAACCTG |
| | | | [13] | GACCTGCCGTCTAGAAAAACCTGCC |
| | | | [14] | TGCCGTCTAGAAAAACCTGCCAAAT |
| | | | [15] | CCGTCTAGAAAAACCTGCCAAATAT |
| | | | [16] | GTCTAGAAAAACCTGCCAAATATGA |

The annotations of the selected mRNA probes are given in Table 20. The annotations were obtained from Bioconductor package hu35ksuba.db (Marc Carlson, Seth Falcon, Herve Pages and Nianhua Li (2008). hu35ksuba.db: Affymetrix Human Genome HU35K Set annotation data (chip hu35ksuba). R package version 2.2.3.) in combination with the information available via PubMed [http://www.ncbi.nlm.nih.gov/pubmed/].
Table 20

**Table 20: Annotation of mRNA probes selected during LOO cross validation (1^{st} column is SEQ-ID-No)**

| | | | | |
|---|---|---|---|---|
| 268 | X01677_s_at | X01677 | NM_002046.3 | Hs.544577 |

### Example2.3: mRNA and microRNA, prostate cancer

We use the prostate cancer data of Ramaswamy et al. (2001) [Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. Multiclass cancer diagnosis using tumor gene expression signatures. Proc Natl Acad Sci USA. 2001;98(26):15149-54] and Lu et al. (2005) [Lu J, Getz G, Miska EA, Alvarez-Saavedra E, Lamb J, Peck D, Sweet-Cordero A, Ebert BL, Mak RH, Ferrando AA, Downing JR, Jacks T, Horvitz HR, Golub TR. MicroRNA expression profiles classify human cancers. Nature. 2005;435(7043):834-8] to develop a multilevel classifier using mRNA and microRNA data. The data are available from the home page of the Braoad Institute[see http://www.broad.mit.edu/publications/broad900 and http://www.broad.mit.edu/publications/broad993s]. Overall the mRNA and microRNA data of six normal tissues and six tumor tissues are available. The hybridisations were done with a bead-based array containing microRNA probes as well as with the Affymetrix HU6800 and HU35KsubA array for measuring the mRNA. We used only the mRNA data of the HU6800 arrays.

### Analysis:

For developing and validating a classifier based on these data we used linear discriminant analysis in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), neural networks (NN), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.

The low level analysis consisted of the variance stabilizing transformation of Huber et al (2002) (often called normalization) in case of the microRNA as well as of the mRNA data. Again there is a large number of alternative methods which could be used. Several examples are given in Cope et al. (2004) or Irizarry et al. (2006) In each cross validation step we selected those two normalized microRNA probes, respectively those four normalized mRNA probes for classification which had the largest median of pairwise differences (in absolute value) beyond those microRNA probes with p value equal or smaller than 0.01 by the Mann-Whitney test. This is, we used a so called ranker for feature selection. Again there are numerous other feature selection strategies we could have used, some examples are given in Hall et al. 2003. Overall a microRNA, respectively mRNA probe may have been chosen up to twelve times due to LOO cross-validation.

Using only microRNA data we obtain the estimated errors given in Table 21
Table 21

**Table 21: microRNA data, classification error via LOO cross validation**

| **classifier vs. true** | **prostate cancer** | **Normal** |
|---|---|---|
| **prostate cancer** | 83.3% | 0.0% |
| **normal** | 16.7% | 100.0% |

The estimated overall accuracy using LOO cross-validation is 91.7 %. Sensitivity, specificity, positive and negative predictive value are 83.3%, 100%, 100% and 85.7%, respectively. In a second step we used the mRNA data of the HU6800 array. The results can be read off from Table 22. We get an estimated overall accuracy of 75.0 % again using LOO cross-validation. Sensitivity, specificity, positive and negative predictive value are 83.3%, 66.7%, 71.4% and 80.0%, respectively.
Table 22

**Table 22: mRNA data, classification error via LOO cross validation**

| **classifier vs. true** | **prostate cancer** | **Normal** |
|---|---|---|
| **prostate cancer** | 83.3% | 33.3% |
| **normal** | 16.7% | 66.7% |

In the last step we combine microRNA and mRNA data and obtain the results given in Table 23. That is, the estimated overall accuracy using cross-validation is 91.7 %. Sensitivity, specificity, positive and negative predictive value are 100.0%, 83.3%, 85.7% and 100.0%, respectively. Hence, this combination increases the sensitivity (correct classification of cancer samples) from 83.3 % to 100.0 % and negative predictive value form 85.7%, respectively 80.0% to 100.0%.
Table 23

**Table 23: microRNA and mRNA data, classification error via LOO cross validation**

| **classifier vs. true** | **prostate cancer** | **Normal** |
|---|---|---|
| **prostate cancer** | 100.0% | 16.7% |
| **normal** | 0.0% | 83.3% |

The microRNA probes which were selected during cross-validation are given in Table 24.
Table 24

**Table 24: microRNA probes selected during LOO cross validation (1^{st} column is SEQ-ID-No)**

| | | | |
|---|---|---|---|
| 735 | hsa-miR-206 | 2 | CCACACACTTCCTTACATTCCA |

The results of the Sanger sequence search according to Griffiths-Jones et al. (2008) for known human microRNAs are given in Table 25
Table 25

**Table 25: Results of the Sanger sequence search 1 for known human microRNAs for microRNA probes selected during LOO cross validation (1^{st} column is SEQ-ID-No)**

| | | | |
|---|---|---|---|
| 738 | hsa-miR-206 | hsa-mir-206 | UGCUUCCCGAGGCCACAUGCUUCUUUAUCCCCAUA UGGAUUACUUUGCUAUGGAAUGUAAGGAAGUGUGUG GUUUCGGCAAGUG |

The mRNA probes which were selected during cross-validation are given in Table 26. The probe sequences were obtained from Bioconductor package hu6800probe [The Bioconductor Project, www.bioconductor.org (2008). hu6800probe: Probe sequence data for microarrays of type hu6800. R package version 2.2.0].
Table 26

**Table 26: mRNA probes selected during LOO cross validation**

| | | | | |
|---|---|---|---|---|
| 833-852 | S82297_at | 2 | [1] | GCTATCCAGCATTCAGGTTTACTCA |
| | | | [2] | ATCCTGAAGCTGACAGCATTCGGGC |
| | | | [3] | CCTGAAGCTGACAGCATTCGGGCCG |
| | | | [4] | AAGCTGACAGCATTCGGGCCGAGAT |
| | | | [5] | GCTGACAGCATTCGGGCCGAGATGT |
| | | | [6] | TGACAGCATTCGGGCCGAGATGTCT |
| | | | [7] | CATTCGGGCCGAGATGTCTCGCTCC |
| | | | [8] | GGCCGAGATGTCTCGCTCCGTGGCC |
| | | | [9] | GGAGGTTTGAAGATGCCGCAGGATC |
| | | | [10] | GAGATGTCTCGCTCCGTGGCCTTAG |
| | | | [11] | GATGTCTCGCTCCGTGGCCTTAGCT |
| | | | [12] | TGTCTCGCTCCGTGGCCTTAGCTGT |
| | | | [13] | CGTGGCCTTAGCTGTGCTCGCGCTA |
| | | | [14] | CTTAGCTGTGCTCGCGCTACTCTCT |
| | | | [15] | TAGCTGTGCTCGCGCTACTCTCTCT |
| | | | [16] | GCTGTGCTCGCGCTACTCTCTCTTT |
| | | | [17] | TGTGCTCGCGCTACTCTCTCTTTCT |
| | | | [18] | GCCTGGAGGCTATCCAGCATTCAGG |
| | | | [19] | CTGGAGGCTATCCAGCATTCAGGTT |
| | | | [20] | GGAGGCTATCCAGCATTCAGGTTTA |
| 873-892 | J02611_at | 1 | [1] | TGAGAAGATCCCAACAACCTTTGAG |
| | | | [2] | GATCCCAACAACCTTTGAGAATGGA |
| | | | [3] | CTTTGAGAATGGACGCTGCATCCAG |
| | | | [4] | ACGCTGCATCCAGGCCAACTACTCA |
| | | | [5] | CATCCAGGCCAACTACTCACTAATG |
| | | | [6] | TTCCTGGTTTATGCCATCGGCACCG |
| | | | [7] | GTTTATGCCATCGGCACCGTACTGG |
| | | | [8] | GATCCTGGCCACCGACTATGAGAAC |
| | | | [9] | GGCCACCGACTATGAGAACTATGCC |
| | | | [10] | TGAGAACTATGCCCTCGTGTATTCC |
| | | | [11] | CCTCGTGTATTCCTGTACCTGCATC |
| | | | [12] | GTATTCCTGTACCTGCATCATCCAA |
| | | | [13] | CTGTACCTGCATCATCCAACTTTTT |
| | | | [14] | CTGCATCATCCAACTTTTTCACGTG |
| | | | [15] | TGCTTGGATCTTGGCAAGAAACCCT |
| | | | [16] | CACAGACCAGGTGAACTGCCCCAAG |
| | | | [17] | CCAGGTGAACTGCCCCAAGCTCTCG |
| | | | [18] | AGGTTCTACAGGGAGGCTGCACCCA |
| | | | [19] | ACTCCATGTTACTTCTGCTTCGCTT |
| | | | [20] | CCTGTTACCTTGCTAGCTGCAAAAT |

The annotations of the selected mRNA probes are given in Table 27. The annotations were obtained from Bioconductor package hu6800.db [Marc Carlson, Seth Falcon, Herve Pages and Nianhua Li (2008). hu6800.db: Affymetrix HuGeneFL Genome Array annotation data (chip hu6800). R package version 2.2.3.] in combination with the information available via PubMed [http://www.ncbi.nlm.nih.gov/pubmed/].
Table 27

**Table 27: Annotation of mRNA probes selected during LOO cross validation (1^{st} column is SEQ-ID-No)**

| | | | | |
|---|---|---|---|---|
| 900 | J02611_at | J02611 | NM_001647.2 | Hs.522555 |

### Example 3: Metabolites and mRNA: Ischemia/Hypoxia

### Ischemia and hypoxia

Early diagnosis will buy critical time for timely intervention and selection of the appropriate therapy and thus to prevent fatal permanent brain damage

As for infants, in industrial countries the percentage of preterm subjects has increased during the last decades and now risen up to 12% of all live births [Martin JA, Hamilton BE, Sutton PD et al. Births: final data for 2004. Natl Vital Stat Rep. 2006; 55:1-101; Martin JA, Hamilton BE, Sutton PD et al. Births: final data for 2005. Natl Vital Stat Rep. 2007; 56:1-103].

However, developmental brain injury and the subsequent neurological sequelae are still a major personal burden for affected individuals and their families and constitutes a considerable socioeconomic problem.

Early detection of a status of ischemia/hypoxia or stroke in man or of perinatal brain lesions in adult patients and preterm infants will enable and the application of successful therapeutic regimens and allow to control the consequences of these measures.

We use the ischemia data obtained from a rat hypoxia model to develop a multi-level classifier using metabolite data from brain samples and qPCR data from plasma.

### Animal model

A model of HI brain injury based on Rice-Vanucci's procedure was performed at postnatal day 7 (P7) [Rice JE, III, Vannucci RC, Brierley JB. The influence of immaturity on hypoxic-ischemic brain damage in the rat. Ann Neurol. 1981; 9:131-141] Sprague-Dawley rat pups (from Charles River, Wilmington, MA, U.S.A.) of either sex were randomly assigned a) the experimental groups and b) the time. For operation animals were anesthetized with inhaled isoflurane 3% in 02, the right carotid artery was accessed through a midline incision and surgical ligation was performed with a double suture and a permanent incision. The procedure was performed at room temperature (23-25°C). After closure of the neck wound, pups were returned to their dams for 2 h. The entire surgical procedure lasted no longer than 10 min. The pups were then exposed to hypoxia at 8% oxygen for 100 minutes. Adequate measures were taken to minimize pain and discomfort, complying with the European Community guidelines for the use of experimental animals. The study protocol was approved by the Austrian committee for animal experiments.

Sham-operated animals underwent anesthesia, neck incision and vessel manipulation without ligation or hypoxia. Control animals were kept without any damage. Animals were euthanized i) immediately after hypoxia (P7), ii) after 24hrs (P8), iii) after 5 days (P12), brains were collected, rinsed with PBS and immediately frozen in liquid nitrogen and stored at -70°C until further preparation.

### Sample preparation

Brain samples were thawed on ice for 1 hour and homogenates were prepared by adding PBS-buffer (phosphate buffered saline, 0.1 µmol/L; Sigma Aldrich, Vienna, Austria) to tissue sample, ratio 3:1 (w/v), and homogenized with a Potter S homogenizer (Sartorius, Goettingen, Germany) at 9 g on ice for 1 minute. To enable analysis of all samples in one batch, samples were frozen again (-70°C), thawed on ice (1 h) on the day of analysis and centrifuged at 18000 g at 2°C for 5 min. All tubes were prepared with 0.001% BHT (butylated hydroxytoluene; Sigma-Aldrich, Vienna, Austria) to prevent autooxidation [Morrow, J. D. and L. J. Roberts. Mass spectrometry of prostanoids: F2-isoprostanes produced by non-cyclooxygenase free radical-catalyzed mechanism. Methods Enzymol. 233 (1994): 163-74]. Overall the data obtained from nine control and seven ischemic animal samples were processed. The metabolite concentrations were measured using a commercial Kit (Marker *IDQ*^{™}, Biocrates AG, Innsbruck, Austria) as well as other mass-spectroscopy based methods described below.

Extracted samples were analyzed by a new developed online solid phase extraction liquid chromatography tandem mass spectrometry method (online SPE-LC-MS/MS). All procedures (sample handling, analytics) were performed by co-workers blinded to the groups. For simultaneous quantitation of free prostaglandins and lipoxygenase derived fatty acid metabolites in brain homogenates we used a LC-MS/MS based method as described by Unterwurzacher et al. [Unterwurzacher I, Koal T, Bonn GK et al. Rapid sample preparation and simultaneous quantitation of prostaglandins and lipoxygenase derived fatty acid metabolites by liquid chromatography-mass spectrometry from small sample volumes. Clin Chem Lab Med. 2008; 46:1589-1597] for brain tissue. Due to matrix effects observed during analysis of brain samples, an online solid phase extraction (SPE) step was implemented prior to chromatographic separation using a C18 Oasis HLB column (2.1 x 20 mm, 25 µm particle size; Waters, Vienna, Austria) as online SPE column. The quantification of the metabolites in the extracted biological sample is achieved by reference to appropriate internal standards and by use of the most sensitive and selective electrospray ionization (ESI) multiple reaction monitoring (MRM) MS/MS detection mode. The method was validated for tissue samples homogenates according the "Guidance for Industry - Bioanalytical Method Validation", U.S. Department of Health and Human Services, Food and Drug Administration, 2001. For the online SPE-LC-MS/MS analysis 20 µL of the extracted homogenate was injected.

### RNA extraction and cDNA synthesis:

The two divided brain hemispheres of newborn RNU rats were collected in 1 ml TRIzol Reagent (Invitrogen Life Technologies, Austria), frozen in liquid nitrogen and stored at -80°C until further processing. The RNA extraction was done according to manufacturer's instructions. Briefly, the brain hemispheres were homogenized in TRlzol on ice using a micropistill. After complete homogenization a chloroform extraction step resulting in an RNA containing aqueous phase, followed by precipitation with isopropyl alcohol was affiliated. After two washing steps with 75% ethanol the briefly air dried RNA was resuspended in DEPC-treated water, the RNA concentration was determined using an UV-spectrophotometer (Ultrospec 3300 pro, Amersham, USA) and stored at -80°C until processing for cDNA synthesis.

Prior to reverse trancription (RT) an amount of 1µg of total RNA was treated with DNase I, RNase-free (Deoxyribonuclease I, Fermentas, Germany) according to manufacturer's instructions to remove potential contaminating DNA. After DNase I treatment the samples were processed for cDNA synthesis using the RevertAid M-MuLV reverse transcriptase (Fermentas, Germany). Each reaction consisted of 5x RT-reaction buffer, 10 mM deoxyribonucleotide triphosphate mixture (dNTPs), 0,2 µg/µl random hexamer primer, an RNase inhibitor and the RevertAid M-MuLV-RT (all from Fermentas, Germany). Samples were incubated at 25°C for 10 minutes followed by 60 minutes at 42°C in a waterbath. The reaction was terminated by heating to 70°C for 10 minutes followed by chilling on ice. The cDNA samples were stored at -20°C until processing for quantitative real-time PCR using the BioRad iCycler iQ. The cDNA samples were prediluted 1:10 before used as template for quantitative real-time PCR.

### Quantitative real-time PCR (q-RT-PCR):

The quantitative real-time PCR was carried out in 96-well 0,2 ml thin-wall PCR plates coverd with optically clear adhesive seals (BioRad Laboratories, Austria) in a total volume of 25 µl. The real-time PCR reaction mixture consisted of 1x iQ SYBR Green Supermix (BioRad Laboratories, Austria), 0,4 µM of each gene specific primer and 5 µl of prediluted cDNA. Initially the mixture was heated to 95°C for 3 minutes to activate the iTaq DNA polymerase, followed by 45 cycles consisting of denaturation at 95°C for 20 seconds and annealing at 60°C for 45 seconds. After the amplification a melting curve analysis was added to confirm PCR product specificity. No signals were detected in the no-template controls.

The results were analysed using the iCycler iQ5 Optical System Software Version 2.0 (BioRad Laboratories, Austria). The baseline was manually set and the threshold automatically by the software.

The crossing point of the amplification curve with the threshold line represents the cycle threshold (ct). All samples were run in triplicates and the mean value was used for further calculations.

During the optimization process all gene specific primer pairs were run in a gradient PCR to determine the optimal annealing temperature, the PCR products were loaded on a 2% agarose gel containing ethidium bromide to confirm specificity of the amplification product and the absence of primer dimer formation.

The sequence of gene specific primer pairs used are given in Table 28 (1^{st} column is SEQ-ID-No).
Table 28

**Table 28: Metabolite data, classification error via LOO cross validation**

| | | | |
|---|---|---|---|
| 901 | rSDF1a-LC1 | 181 bp | 5'-AGTGACGGTAAGCCAGTCAG-3' |
| 902 | rSDF1a-LC2 | | 5'-TCCACTTTAATTTCGGGTCA-3' |
| 903 | rVEGF-LC1 | 195 bp | 5'-GAAAGGGAAAGGGTCAAAAA-3' |
| 904 | rVEGF-LC2 | | 5'-CACATCTGCAAGTACGTTCG-3' |
| 905 | rACTB-LC1 | 160 bp | 5'-AAGAGCTATGAGCTGCCTGA-3' |
| 906 | rACTB-LC2 | | 5'-TACGGATGTCAACGTCACAC-3' |

### Analysis of qPCR and Metabolomics data:

For developing and validating a classifier based on these data we used support vector machines [Schöllkopf, B. and Smola, A. (2001) Learning with Kernels: Support Vector Machines, Regularization, Optimization, and Beyond. MIT Press, Cambridge] in combination with leave-one-out (LOO) cross-validation where each analysis step - including low level analysis - was repeated in each cross-validation step. This is one possibility. Of course, we could also have used a split-sample, a bootstrap or a different k-fold (k not equal to 1) cross-validation approach. Moreover, we could have used a different class of functions for classification e.g. logistic regression, (diagonal) linear or quadratic discriminant analysis (LDA, QDA, DLDA, DQDA), shrunken centroids regularized discriminant analysis (RDA), random forests (RF), support vector machines (SVM), generalized partial least squares (GPLS), partitioning around medoids (PAM), self organizing maps (SOM), recursive partitioning and regression trees, K-nearest neighbor classifiers (K-NN), bagging, boosting, naïve Bayes and many more.

The low level analysis consisted of a variance stabilizing transformation via the binary logarithm (i.e., log to base 2) for the metabolite data In each cross validation step we selected those four normalized metabolites, which had the largest differences (in absolute value) of the mean values beyond those probes with p value equal or smaller than 0.1 by the Welch t-test. This is, we used a so called ranker for feature selection. Again there are numerous other feature selection strategies we could have used, some examples are given in Hall et al. 2003 Overall a metabolite may have been chosen up to 16 times due to LOO cross-validation.

Using only metabolomics data we obtain the estimated errors given in Table 29.
Table 29

**Table 29: Metabolite data, classification error via LOO cross validation**

| **classifier vs. true** | **ischemia** | **control** |
|---|---|---|
| **Ischemia** | 57.1% | 33.3% |
| **Control** | 42.9% | 66.7% |

The estimated overall accuracy using LOO cross-validation is 62.5 %, sensitivity is 57.1%, specificity is 66.7%, positive predictive value is 57.1% and negative predictive value is 66.7%. In a second step we used qPCR data obtained for SDF1 and VEGF. The PCR data was normalized via the reference gene Actin-beta. The classification results can be read off from Table 30. We get an estimated overall accuracy of 68.9 % again using LOO cross-validation. The estimated values for sensitivity, specificity, positive and negative predictive value are 57.1%, 77.8%, 66.7% and 70.0%, respectively.
Table 30

**Table 30: qPCR data, classification error via LOO cross validation**

| **classifier vs. true** | **ischemia** | **normal** |
|---|---|---|
| **Ischemia** | 57.1% | 22.2% |
| **Normal** | 42.9% | 77.8% |

In the last step we combine metabolite and qPCR data and obtain the results given in Table 31. That is, the estimated overall accuracy using cross-validation is 75.0 %. Hence, this combination increases the overall accuracy from 62.5 % resp. 68.9% to 75.0 %. Sensitivity, specificity, positive and negative predictive value are 71.4%, 77.8%, 71.4% and 77.8%, respectively. Hence, beside overall accuracy, sensitivity as well as positive and negative predictive value are enhanced.
Table 31

**Table 31: Metabolite and qPCR data, classification error via LOO cross validation**

| **classifier vs. true** | **ischemia** | **normal** |
|---|---|---|
| **Ischemia** | 71.4% | 22.2% |
| **Normal** | 18.6% | 77.8% |

The metabolites which were selected during cross-validation are given in Table 32.
Table 32

**Table 32: Metabolites selected during LOO cross validation**

| **Nr.** | **Metabolite** | **Times selected** | **Comments** |
|---|---|---|---|
| 1 | GLEN-ETC | 16 | PTC = Phenylthiocarbamoyl |
| 2 | xLeu-PTC | 15 | |
| 3 | Ala-PTC | 11 | |
| 4 | 12S-HETE | 8 | = 12(S)-Hydroxyeicosatetraenoic acid |
| 5 | Alanine | 4 | |
| 6 | xLeucine | 3 | |
| 7 | DHA | 3 | = Decosahexaenoic acid |
| 8 | Ser-PTC | 2 | |
| 9 | Glu | 1 | |
| 10 | Glutamic Acid | 1 | |

In Table 32, the total of times selected must be 64, wherein each individual metabolite might be selected a maximum of 16 times.
Table 33

**Table 33: Metabolite data, classification error via LOO cross validation (1^{st} column is SEQ-ID-**

| | | | |
|---|---|---|---|
| 265 | ACTB | NM_001101.2 | Hs.520640 |
| | | | Hs.708120 |

### Examples

In one example, first, a biological sample from a subject in need of diagnosis, or response or survival prognostication is obtained. Second, an amount of a RNA, microRNA, peptide or protein, metabolite is selected and is measured from the biological sample. Third, the amount of RNA, microRNA, peptide or protein, metabolite, is detected in the sample and is compared to either a standard amount of the respective biomolecule present in a normal cell or a non-cancerous cell or tissue or plasma, or an amount of the RNA, microRNA, peptide or protein, metabolite is present in the control sample. If the amount of RNA, microRNA, peptide or protein, metabolite in the sample is different to the amount of RNA, microRNA, peptide or protein, metabolite in the standard or control sample, the processing and classification of concentration data and classifier generation as described before (Table 1) from at least two groups/species of biomolecules comprising RNA, microRNA, peptide or protein, metabolites affords a value or score assigned to a diseased state with some probability then the subject is diagnosed as having cancer, the prognosis is a low expected response to the cancer treatment, or the prognosis is a low expected survival of the subject. The prognoses are relative to a subject with cancer having normal levels of the RNA, microRNA, peptide or protein, metabolite or relative to the average expected response or survival of a patient having a complex disease. It is clear that these complex diseased states can also be due to intoxication and drug abuse.

Another example comprises the following steps. First, a biological sample containing RNA, microRNA, peptide or protein, metabolite is obtained from the subject. The biological sample is reacted with a reagent capable of binding to an RNA, microRNA, peptide or protein, metabolite. The reaction between the reagent and the microRNA forms a measurable RNA, microRNA, peptide or protein, metabolite product or complex. The measurable RNA, microRNA, peptide or protein, metabolite product or complex is measured, the data processed to afford a score applying the steps as specified under Fig. 1 and then compared to either the standard or the control score value.

The examples include the analysis and classifier generation from quantitative data of the aforementioned types of biomolecules obtained from different, distinct tissues from one individual and show that this is advantageous in recognizing distinct states related to complex diseases as data from different sites of an affected organism contribute to biomarker/classifier description. of developing a complex diseases in the sense of the present invention.

Samples can be obtained in any manner known to a skilled Samples can be obtained in any manner known to a skilled artisan. The sample optimally can include tissue believed to be cancerous, such as a portion of a surgically removed tumor but also blood containing cancer cells. However, the examples are not limited to just tissue believed to be altered (with regard to concentrations of biomolecules such as RNA, micro RNA, protein, peptide, metabolites) due to a complex disease. Instead, samples can be derived from any part of the subject containing at least some tissue or cells believed to be affected by the complex disease, in particular, cancer and/or having being exposed or in contact to cancer tissue or cells or by contact to body liquids such as blood distributing certain biomolecules within the body.

Another example of a method of quantifying RNA or microRNA is as follows: hybridizing at least a portion of the RNA or microRNA with a fluorescent nucleic acid, and reacting the hybridized RNA or microRNA with a fluorescent reagent, wherein the hybridized RNA or microRNA emits a fluorescent light. Another method of quantifying the amount of RNA or microRNA in a sample is by hybridizing at least a portion the RNA or microRNA to a radiolabeled complementary nucleic acid. In instances when a nucleic acid capable of hybridizing to the RNA or microRNA is used in the measuring step, in case of the microRNA the nucleic acid is at least 5 nucleotides, at least 10 nucleotides, at least 15 nucleotides, at least 20 nucleotides, at least 25 nucleotides, at least 30 nucleotides or at least 40 nucleotides; and may be no longer than 25 nucleotides, no longer than 35 nucleotides; no longer than 50 nucleotides; no longer than 75 nucleotides, no longer than 100 nucleotides or no longer than 125 nucleotides in length. The nucleic acid is any nucleic acid having at least 80% homology, 85% homology, 90% homology, 95% homology or 100% homology with any of the complementary sequences for the microRNAs. A suitable RNA parameter, e.g. is the amount of RNA or microRNA which is compared to either a standard amount of the RNA or microRNA present in a normal cell or a non-cancerous cell, or to the amount of RNA or microRNA in a control sample. The comparison can be done by any method known to a skilled artisan. An example of comparing the amount of the RNA or microRNA in a sample to a standard amount is comparing the ratio between 5S rRNA and the RNA or microRNA in a sample to a published or known ratio between 5S rRNA and the RNA or microRNA in a normal cell or a non-cancerous cell. An example of comparing the amount of microRNA in a sample to a control is by comparing the ratios between 5 S rRNA and the RNA or microRNA found in the sample and in the control sample. In instances when the amount of RNA or microRNA is compared to a control, the control sample may be obtained from any source known to have normal cells or non-cancerous cells. Preferably, the control sample is tissue or body fluid from the subject believed to be unaffected by the respective complex disease contain only normal cells or non-cancerous cells.

Measuring the amount of RNA, microRNA, peptide or protein, metabolite can be performed in any manner known by one skilled in the art of measuring the quantity of RNA, microRNA, peptide or protein within a sample. An example of a method for quantifying RNA or microRNA is quantitative reverse transcriptase polymerase chain reaction, PCR or quantitation and relative quantitation applying sequencing or second generation sequencing.

Protein measurement, absolute and relative protein quantitation of individual protein species as well as quantitation of metabolites within a tissue or in a preparation of cells can be performed applying Western blotting, Enzyme Linked Immunoassay (ELISA) Radioimmunoassay or other assays utilizing antibodies or other protein binding molecules, mass spectrometry for protein or peptide identification, quantitation or relative quantitation using MALDI, Electrospray or other types of ionisation, protein and antibody arrays employing antibodies or other molecules binding proteins such as aptamers. The compound capable of binding to RNA, microRNA, peptide or protein and metabolite can be any compound known to a skilled artisan as being able to bind to the RNA, microRNA, peptide or protein in a manner that enables one to detect the presence and the amount of the molecule. An example of a compound capable of binding RNA, microRNA, peptides or proteins as well as low molecular weight compounds and metabolites is a nucleic acid capable of hybridizing or an aptamer capable of binding to nucleic acids, RNA, microRNA, proteins and peptides. The nucleic acid preferably has at least 5 nucleotides, at least 10 nucleotides, at least 15 nucleotides, at least 20 nucleotides, at least 25 nucleotides, at least 30 nucleotides, at least 40 nucleotides or at least 50 nucleotides. The nucleic acid is any nucleic acid having preferably at least 80% homology, 85% homology, 90% homology, 95% homology or 100% homology with a sequence complementary to an RNA or microRNA , which also might be derived from corresponding DNA data, or an aptamer capable of binding RNA, microRNA, peptide or protein or metabolite. One specific example of a nucleic acid capable of binding to RNA or microRNA is a nucleic acid primer for use in a reverse transcriptase polymerase chain reaction.

The binding of the compound to at least a portion of the RNA, microRNA, peptide or protein and metabolite forms a measurable complex. The measurable complex is measured according to methods known to a skilled artisan. Examples of such methods include the methods used to measure the amount of the RNA, microRNA, peptide or protein, metabolite employed in the inventive method discussed above.

If there is an increased or decreased level of measurable complex relative to a standard amount of RNA, microRNA, peptide or protein found in a normal or a non-cancerous cell, or in a control sample, then the sample either contains a pre-cancerous cell or cancer cell, thereby being diagnostic of a cancer; prognosticates an expected response to a cancer treatment; or prognosticates an expected survival of the subject.

The composition can comprise a compound capable of binding to at least a portion of RNA, microRNA, peptide, protein or metabolite selected from the group consisting of RNA, microRNA, peptide or protein, metabolite. The composition comprises a compound capable of binding to at least a portion of a RNA, microRNA, peptide or protein selected from the group consisting of molecules summarized in the described examples and the lists of molecules and binding probes binding these endogenous biomolecules but is not limited to that. The various examples described above demonstrate that the method generally functions with a composition of 2-4 types of the defined biolmolecules, proteins or peptides, RNA, microRNA (i.e. RNA plus microRNA, RNA plus protein, protein plus microRNA, RNA plus protein plus microRNA, and a combination of these biomolecules and combinations of biomolecules with metabolites, selected and combined from various experiments investigating tissue from a subject having a complex disease with a performance which is superior than that of a test or diagnostic or prognostic tool comprising a set of preselected biomolecules composed of just one type such as RNA, protein, metabolite or microRNA solely.

Another example is a composition comprising a second compound capable of binding to a RNA, microRNA, peptide or protein and metabolite that is different from the RNA, microRNA, peptide or protein, metabolite that the first compound is capable of binding. Another example is a composition comprising a third compound capable of binding to a RNA, microRNA, peptide or protein, metabolite that is different from the RNA, microRNA, peptide or protein, metabolite that the first and second compounds are capable of binding.

An exemplary method evaluates candidate therapeutic agents. The method can be applied to identify molecules that modulate the concentrations of one to several of the mentioned biomolecules assigned to at least two or more of the stated molecule classes; RNA, microRNA, peptide/proteins, metabolites. Alternatively, assays may be conducted to identify molecules that modulate the activity of a protein encoded by a gene. Another aspect is a kit for diagnosing, or prognosticating a complex disease. In one example, the kit is for diagnosing a subject with a complex disease.

Another example is a kit for prognosticating a a complex disease, wherein the prognosis is an expected response by a subject to a treatment of the a complex disease. In another embodiment of this aspect, the kit is for prognosticating a a complex disease, wherein the prognosis is an expected survival of a subject with a complex disease. The kit comprises a composition capable of binding to at least a portion of a RNA, microRNA, peptide or protein, metabolite with increased or decreased concentration, over- or under-expressed in a cancer cell, wherein the RNA, microRNA, peptide or protein, metabolite is selected from-but not limited to the group consisting of the molecules listed in the examples outlined above or binding to the binding probes or determined quantitatively by methods described in the examples above and and wherein the differential expression (over-expression or under-expression or the concentration changes of several molecules out of RNA, microRNA, peptide or protein, metabolites in a combination of at least molecules from 2 different biomolecule classes (RNA plus microRNA, RNA plus proteins or peptides, microRNA plus protein or peptides, RNA plus microRNA plus proteins or peptides and combinations of all these with metabolites), comprising, but not limited, to the classes of compounds, the described binding probes, the agents and sequences specified in the described examples is diagnostic for a complex disease, or prognosticates the expected response or survival of the subject. The binding of the nucleic acid or aptamer or antibody to the target RNA, microRNA, peptide or protein, and or metabolite is diagnostic for a complex disease, prognosticates an expected response to a treatment, or prognosticates an expected survival of a subject having a complex disease.

The isolated RNA, microRNA, peptide or protein, metabolite can be associated with known diagnostic tools, such as protein chips, antibody chips, aptamer chips, DNA or RNA chips with various modes of detection of binding including but not limited to detection by use of fluorophores, electrochemical detection or transfer of an chemical signal to a change of electrical current, resistance or charge, RNA probes, or RNA primers.

The sequence listing accompanying the present application comprising sequences with SEQ-IDs No 1 to is SEQ-IDs No 908 is part of the disclosure of the present invention.

## Claims

1. A method for in vitro diagnosing a complex disease namely acute myeloid leukemia, AML, in at least one biological sample of at least one tissue of a mammalian subject, wherein said mammalian subject is a human being, and said biological sample is/are blood and/or blood cells and/or bone marrow, comprising the steps of:
a) measuring microRNA expression levels and CD34 presence in said sample and storing the obtained set of values as raw data in a database;
b) mathematically preprocessing said raw data in order to reduce technical errors being inherent to the measuring procedures used in step a), wherein raw data of microRNA expression are preprocessed using a variance-stabilizing normalization and summarizing the normalized multiple probe signals to a single expression value, using the median and after preprocessing step b) a further step of feature selection is inserted, wherein a ranker, in particular a Mann-Whitney significance test combined with largest median pairwise differences, as filter for microRNA expression data is used,
c) selecting a logistic regression classifier algorithm;
d) said classifier algorithm of step c) being trained on at least one training data set containing preprocessed data from subjects being divided into classes according to their AML-types, in order to select a classifier function to map said preprocessed data to said AML-types, wherein the training data set of the classifier algorithm includes preprocessed and filtered microRNA expression data and CD34 presence, and the training is carried out with an n-fold cross-validation;
e) applying said trained logistic regression classifier algorithm of step d) to predict the class label of said data set of step b) in order to diagnose the condition of the subject, namely a specific AML-type.

2. Method according to claim 1, **characterized in that** said tissue is selected from the group consisting of blood bone marrow tissue, and/or said sample is a biopsy sample.

3. Method according to anyone of claims 1 or 2, wherein the said n-fold cross-validation according to step d.) is 5 to 10-fold.

4. Method according to claim 1, **characterized in that** the following DNA probes for targeting said microRNA are used: Seq-ID No. 1 to Seq-ID No. 14; and/or the following microRNA-target sequences are used: Seq-ID Nos. 15 to 26.

5. Use of a kit for carrying out a method for in vitro diagnosing a complex disease namely acute myeloid leukemia, AML, according to claim 1, in at least one biological sample of at least one tissue of a mammalian subject, wherein said mammalian subject is a human being, and said biological sample is/are blood and/or blood cells and/or bone marrow, comprising : a) detection agents for the measuring of microRNA expression levels and CD34 presence; b) positive and/or negative controls; and c) classification software for classification of the results achieved with said detection agents.

## Patentansprüche

1. Verfahren für die in-vitro-Diagnostizierung einer komplexen Krankheit, bei der es sich um akute myeloische Leukämie - AML - handelt, in mindestens einer biologischen Probe von mindestens einem Gewebe eines Subjekts aus der Gattung Mammalia, wobei das Subjekt aus der Gattung Mammalia ein Mensch ist und es sich bei der biologischen Probe um Blut und/oder Blutkörperchen und/oder Knochenmark handelt, die folgenden Schritte umfassend:
a) Messen von microRNA-Expressionsniveaus und eines CD34-Vorkommens in der Probe und Speichern des erhaltenen Wertesatzes als Rohdaten in einer Datenbank;
b) mathematisches Vorverarbeiten der Rohdaten, um technische Fehler zu verringern, die den in Schritt a) verwendeten Messverfahren innewohnen, wobei Rohdaten der microRNA-Expression unter Verwendung von varianzstabilisierender Normalisierung und Bilden einer Summe aus den mehreren normalisierten Sondensignalen zum einem einzigen Expressionswert vorverarbeitet werden, wobei unter Verwendung des Medianwerts und nach dem Vorverarbeitungsschritt b) ein weiterer Merkmalsauswahlschritt eingefügt wird, wobei ein Rang-Ermittler, insbesondere ein Mann-Whitney-Signifikanztest in Kombination mit größten paarweisen Unterschieden der Medianwerte als Filter für microRNA-Expressionsdaten verwendet wird,
c) Auswählen eines logistische-Regression-Klassifizierungsalgorithmus;
d) wobei der Klassifizierungsalgorithmus von Schritt c) auf mindestens einen Trainingsdatensatz trainiert wird, der vorverarbeitete Daten von Subjekten enthält, die gemäß ihren AML-Typen in Klassen eingeteilt wurden, um eine Klassifizierungsfunktion auszuwählen, um die vorverarbeiteten Daten auf die AML-Typen abzubilden, wobei der Trainingsdatensatz des Klassifizierungsalgorithmus vorverarbeitete und gefilterte microRNA-Expressionsdaten und CD34-Vorkommen umfasst und das Training mit einer n-fachen Kreuzvalidierung durchgeführt wird;
e) Anwenden des trainierten logistische-Regression-Klassifzierungsalgorithmus von Schritt d), um das Klassen-Label des Datensatzes von Schritt b) vorherzusagen, um den Zustand des Subjekts, nämlich einen spezifischen AML-Typ, zu diagnostizieren.

2. Verfahren nach Anspruch 1, **dadurch gekennzeichnet, dass** das Gewebe aus der Gruppe ausgewählt wird, die aus Blut- Knochenmarkgewebe besteht, und/oder die Probe eine Biopsieprobe ist.

3. Verfahren nach einem der Ansprüche 1 oder 2, wobei die n-fache Kreuzvalidierung gemäß Schritt d) 5- bis 10-fach ist.

4. Verfahren nach Anspruch 1, **dadurch gekennzeichnet, dass** die folgenden DNA-Sonden für das Targeting der microRNA verwendet werden: SEQ ID No. 1 bis SEQ ID No. 14; und/oder die folgenden microRNA-Targetsequenzen verwendet werden: SEQ ID Nos. 15 bis 26.

5. Verwendung eines Kits für die Durchführung eines Verfahrens zur in-vitro-Diagnostizierung einer komplexen Krankheit, bei der es sich um akute myeloische Leukämie, AML, handelt, nach Anspruch 1, in mindestens einer biologischen Probe mindestens eines Gewebes eines Subjekts aus der Gattung Mammalia, wobei das Subjekt aus der Gattung Mammalia ein Mensch ist und es sich bei der biologischen Probe um Blut und/oder Blutkörperchen und/oder Knochenmark handelt, umfassend: a) Nachweismittel für die Messung von microRNA-Expressionsniveaus und CD34-Vorkommen; b) positive und/oder negative Kontrollen; und c) Klassifizierungssoftware für die Klassifizierung der Ergebnisse, die mit den Nachweismitteln erhalten werden.

## Revendications

1. Procédé destiné au diagnostic in vitro d'une maladie complexe, à savoir
la leucémie myéloïde aigue, LMA,
dans au moins un échantillon biologique d'au moins un tissu d'un sujet mammifère, où ledit sujet mammifère est un être humain, et ledit échantillon biologique/lesdits échantillons biologiques est/sont du sang, et/ou des cellules de sang, et/ou de la moelle osseuse, comprenant les étapes de :
a) mesure des niveaux d'expression des micro-ARN et de la présence de la protéine CD34 dans ledit échantillon et stockage de l'ensemble des valeurs obtenues en tant que données brutes dans une base de données ;
b) prétraitement mathématique desdites données brutes dans le but de diminuer les erreurs techniques qui sont inhérentes aux protocoles de mesure utilisés dans l'étape a), où les données brutes de l'expression des micro-ARN sont prétraitées en utilisant une normalisation et stabilisation de la variance et en faisant une synthèse des signaux multiples des sondes normalisés en une valeur unique d'expression, en utilisant la médiane et après l'étape b) de prétraitement, une nouvelle étape de sélection des caractéristiques est insérée, dans laquelle un test des rangs, en particulier un test de significativité de Mann-Whitney combiné avec les plus grands écarts médians par paire, est utilisé pour filtrer les données de l'expression des micro-ARN,
c) choix d'un algorithme classifieur de régression logistique ;
d) ledit algorithme classifieur de l'étape c) reposant sur au moins un ensemble de données d'apprentissage contenant les données de prétraitement provenant des sujets divisées en classes selon leurs types de LMA, de façon à sélectionner une fonction de classification pour dresser la carte desdites données prétraitées desdits types de LMA, dans laquelle l'ensemble des données d'apprentissage de l'algorithme classifieur comporte les données d'expression de micro-ARD prétraitées et filtrées et la présence de CD34, et l'apprentissage est réalisé par la méthode de validation croisée globale sur l'ensemble n;
e) application dudit algorithme classifieur de régression logistique d'apprentissage de l'étape d) pour prédire l'étiquette de la classe dudit ensemble de données de l'étape b) de manière à diagnostiquer la condition du sujet, en attribuant un type spécifique de LMA.

2. Procédé selon la revendication 1, **caractérisé en ce que** ledit tissu est sélectionné dans le groupe constitué par les tissus de sang et de moelle osseuse, et/ou ledit échantillon est un échantillon de biopsie.

3. Procédé selon l'une quelconque des revendications 1 ou 2, dans lequel ladite validation croisée globale sur l'ensemble n selon l'étape d) est à effectuer entre 5 et 10 fois.

4. Procédé selon la revendication 1, **caractérisé en ce que** les sondes ADN suivantes sont utilisées pour cibler ledit micro-ARN : Seq-ID N°1 à Seq-ID N°14 ; et/ou les séquences suivantes de micro-ARN cibles sont utilisées : Seq-ID N°15 à 26.

5. Utilisation d'un kit pour réaliser le procédé de diagnostic in vitro de la maladie complexe, à savoir la leucémie myéloïde aigue, LMA,
selon la revendication 1, dans au moins un échantillon biologique d'au moins un tissu d'un sujet mammifère, dans lequel ledit sujet mammifère est un être humain, et ledit échantillon biologique/lesdits échantillons biologiques est/sont le sang et/ou les cellules sanguines et/ou la moelle osseuse, comprenant :
a) les agents de détection pour mesurer les niveaux d'expression de micro-ARN et la présence de CD34 ;
b) des contrôles positifs et/ou négatifs ; et c) un logiciel de classification pour la classification des résultats obtenus par lesdits agents de détection.
